(19) Europäisches Patentamt
European Patent Office
Office européen des brevets

(11) **EP 4 653 424 A1**

(12) **EUROPEAN PATENT APPLICATION**
published in accordance with Art. 153(4) EPC


(43) Date of publication:
**26.11.2025 Bulletin 2025/48**

(21) Application number: **23917320.6**

(22) Date of filing: **26.12.2023**

(51) International Patent Classification (IPC):
***C07D 237/22*** *(2006.01)* ***C07D 403/12*** *(2006.01)*
***A61K 31/4965*** *(2006.01)* ***A61K 31/497*** *(2006.01)*
***A61P 35/00*** *(2006.01)* ***A61P 25/00*** *(2006.01)*

(52) Cooperative Patent Classification (CPC):
**A61K 31/4965; A61K 31/497; A61K 31/50; A61K 31/501; A61P 7/02; A61P 9/00; A61P 9/10; A61P 25/00; A61P 31/00; A61P 35/00; A61P 35/02; A61P 37/00; C07D 237/22; C07D 401/04; C07D 401/12;** (Cont.)

(86) International application number:
**PCT/CN2023/141928**

(87) International publication number:
**WO 2024/152855 (25.07.2024 Gazette 2024/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **17.01.2023 CN 202310057843**



(71) Applicant: **China Pharmaceutical University**
**Nanjing, Jiangsu 210009 (CN)**

(72) Inventors:
- **XU, Yungen**
  **Nanjing, Jiangsu 210009 (CN)**
- **ZOU, Yi**
  **Nanjing, Jiangsu 210009 (CN)**
- **GU, Hongfeng**
  **Nanjing, Jiangsu 210009 (CN)**
- **YANG, Jieping**
  **Nanjing, Jiangsu 210009 (CN)**
- **ZHAO, Xiaolin**
  **Nanjing, Jiangsu 210009 (CN)**
- **YAN, Wenxin**
  **Nanjing, Jiangsu 210009 (CN)**
- **WANG, Chenghao**
  **Nanjing, Jiangsu 210009 (CN)**
- **ZHU, Qihua**
  **Nanjing, Jiangsu 210009 (CN)**

(74) Representative: **Murgitroyd & Company**
**165-169 Scotland Street**
**Glasgow G5 8PL (GB)**


(54) **PYRIDAZINONE PARP INHIBITOR, AND PREPARATION METHOD THEREFOR, AND PHARMACEUTICAL COMPOSITION AND USE THEREOF**


(57) A pyridazinone compound as shown in formula (I), and a preparation method therefor, and a pharmaceutical composition and the use thereof. The compound, as a PARP inhibitor, has potential advantages in terms of blood-brain barrier permeability, achieves inhibitory activity on PARP enzymes at a nanomolar concentration level, and can be used in the preparation of a drug for preventing or/and treating stroke; in addition, the method for preparing the PARP inhibitor is simple and easy.

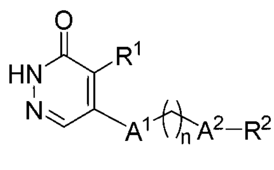

(I)


EP 4 653 424 A1

(52) Cooperative Patent Classification (CPC): (Cont.)
**C07D 403/04; C07D 403/12; C07D 409/12**

## Description

### TECHNICAL FIELD

The present disclosure relates to the field of pharmaceuticals, and particularly, to a pyridazinone derivative, a method for preparing same, a pharmaceutical composition thereof, and use thereof.

### BACKGROUND

Poly(ADP-ribose)polymerase-1 (PARP1) is present in eukaryotic cells and catalyzes the poly-ADP-ribosylation, among other family members. PARP1 is the first known nuclear enzyme with the functionality of catalyzing poly(ADP-ribosyl)phosphate, and other subtypes, such as PARP2, PARP3, PARP4 (VPARP), PARP5a (tankyrase1), PARP5b (tankyrase2), PARP7 (TiPARP), PARP1, were then subsequently isolated.

PARP7 is a gene regulated by AHR and an important member of the PARP family. PARP7 can transfer only one mono-ADP-ribose (MAR), belonging to MonoPARPs. The PARP catalytic domain of PARP7 contains a zinc finger motif that can confer DNA binding capacity and a WWE domain that can mediate protein interactions. The mediated mono-ADP-ribosylation is a reversible post-translational modification involved in a variety of important biological processes, such as immune cell functions, transcriptional regulation, protein expression, and DNA repair.

Furthermore, PARP7 is part of a negative feedback loop that regulates the activity of AHR, which can regulate the immune functions, inflammation and stem differentiation, and play a role in cancers. PARP7 has been demonstrated to be overactive in tumors and plays a key role in cancer cell survival. More importantly, many cancer cells rely on PARP7 for intrinsic cell survival, and studies have shown that PARP7 allows cancer cells to "hide" from the immune system; the inhibition of PARP7 can effectively inhibit the growth of cancer cells, restore interferon signaling, and inhibit the "brakes" of innate and adaptive immune mechanisms. In several cancer models, PARP7 inhibitors exhibited durable tumor growth inhibition, potent anti-proliferative activity, and restoration of interferon signaling.

Stroke is mainly divided into hemorrhagic stroke and ischemic stroke. The former mainly includes cerebral hemorrhage, subarachnoid hemorrhage, and the like; the latter mainly includes transient ischemic attack, cerebral thrombosis, cerebral embolism, and the like. Studies have shown that circular RNAs (circRNAs) are highly expressed in the central nervous system and are involved in the regulation of physiological and pathophysiological processes. Using circRNA microarrays, a significant increase in the circRNA Hect1 (circHect1) level in an ischemic brain tissue was found in a transient middle cerebral artery occlusion (tMCAO) mouse stroke model, and this finding was further validated in acute ischemic stroke (AIS) patient plasma samples. The reduction in circHect1 expression significantly reduced the infarct area, alleviated neuronal defects, and improved the activation of astrocytes in tMCAO mice. Mechanistically, circHect1 acts as an endogenous MIR142 (microRNA 142) sponge to inhibit MIR142 activity, thereby inhibiting PARP7 expression and subsequently inhibiting astrocyte activation through macroautophagy/autophagy. It can be seen from the results that circHect1 and its coupling mechanism are involved in cerebral ischemia, and the inhibition of PARP7 expression can also be used to treat stroke.

Stroke is a common disease in middle-aged and elderly populations, and some survivors may have disability to various extents, even severe disability. Existing drugs are intended to block the neuronal necrosis caused by ischemia, prolong the ischemic tolerance and treatment time window, enhance the neuronal viability, reverse the ischemic semi-dark zone, reduce the infarct volume, and promote the recovery of nerve function in the treatment of stroke, but none of the drugs has yet achieved an accepted therapeutic effect.

RBN-2397 is the first compound with potent inhibitory activity and selectivity for PARP7. However, the compound is only suitable for anti-tumor applications, and the large molecular weight makes it difficult to permeate the blood-brain barrier and thus difficult to apply to central nervous system diseases such as stroke.

### SUMMARY

Objectives: The first objective of the present disclosure is to provide a pyridazinone derivative having inhibitory activity against PARP, the second objective is to provide a method for preparing the compound, the third objective is to provide a pharmaceutical composition comprising the compound, and the fourth objective is to provide use of the compound and the pharmaceutical composition thereof. Technical solution: In order to solve the technical problems in the prior art, the present disclosure provides a compound of formula (I), or a racemate, stereoisomer, tautomer, isotopically labeled form, solvate or pharmaceutically acceptable salt thereof:

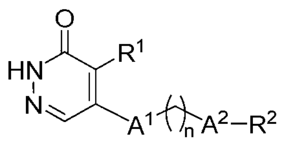

(I)

wherein:

n is selected from 0, 1, 2, 3, and 4;

$R^1$ is selected from hydrogen, deuterium, halogen, cyano, nitro, $C_1$-$C_{12}$ haloalkyl, $C_1$-$C_{12}$ alkyl, $C_1$-$C_{12}$ alkyloxy, $C_1$-$C_{12}$ alkylthio, $C_1$-$C_{12}$ alkylsulfonyl, and -C(O)N($R^{11}$)($R^{12}$); $R^{11}$ and $R^{12}$ are identical or different, and are each independently selected from H, deuterium, and $C_1$-$C_6$ alkyl;

$A^1$ is selected from -O-, -S-,

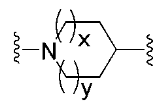

,

and -N($R^3$)-, wherein x and y are each independently selected from 0, 1, and 2; $R^3$ is selected from H, deuterium, $C_1$-$C_{12}$ alkyl, and $C_3$-$C_{14}$ cycloalkyl;

$A^2$ is absent or selected from methylene, vinylene, ethynylene,

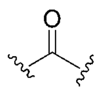 , 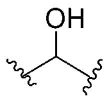 , 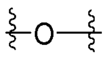 , 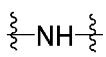 ,

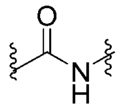

,

and the following groups unsubstituted or optionally substituted with one, two, or more (preferably, one) $R_{a2}$:

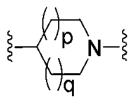

, (

$C_6$-$C_{14}$ aryl, and 5- to 14-membered heteroaryl (preferably, 5- to 14-membered heteroaryl containing one nitrogen atom), wherein p and q are each independently selected from 0, 1, and 2; the $R_{a2}$ groups are identical or different, and are each independently selected from hydrogen, deuterium, halogen, cyano, hydroxy, nitro, $C_1$-$C_{12}$ alkyl, $C_1$-$C_{12}$ alkyloxy, $C_1$-$C_{12}$ haloalkyl, $C_1$-$C_{12}$ haloalkyloxy, -C(O)O$R_{a21}$, -C(O)$R_{a22}$, -N($R_{a23}$)($R_{a24}$), -S(O)2$R_{a25}$, and -S(O)$R_{a26}$; the $R_{a21}$, $R_{a22}$, $R_{a23}$, $R_{a24}$, $R_{a25}$, and $R_{a26}$ groups are identical or different and are each independently selected from H, deuterium, $C_1$-$C_{12}$ alkyl, and $C_1$-$C_{12}$ alkyl-C(O)-;

$R^2$ is selected from the following groups unsubstituted or optionally substituted with one, two, or more (preferably, one or two) $R^{21}$: $C_6$-$C_{14}$ aryl, 5- to 14-membered heteroaryl (preferably, 5- to 14-membered heteroaryl containing one or two nitrogen or sulfur atoms), 3- to 8-membered heterocyclyl (preferably, 3- to 8-membered heterocyclyl containing one nitrogen atom), $C_1$-$C_{12}$ alkyl, -$S(O)_2$-NH-, -C(O)$NH_2$, and $NH_2$-$S(O)_2$-NH-; the $R^{21}$ groups are identical or different, and are each independently selected from hydrogen, deuterium, halogen, cyano, hydroxy, nitro, $C_1$-$C_{12}$ alkyl, $C_1$-$C_{12}$ haloalkyl, $C_6$-$C_{14}$ aryl, 5- to 14-membered heteroaryl (preferably, 5- to 14-membered heteroaryl containing one or two nitrogen or sulfur atoms), 3- to 8-membered heterocyclyl (preferably, 3- to 8-membered heterocyclyl containing one nitrogen atom), $R_{21}$-O-, -C(O)O$R_{22}$, -C(O)$R_{23}$, -N($R_{24}$)($R_{25}$), -$S(O)_2R_{26}$, and -S(O)$R_{27}$; the $R_{21}$, $R_{22}$, $R_{23}$, $R_{24}$, $R_{25}$, $R_{26}$, and $R_{27}$ groups are identical or different, and are each independently selected from H, deuterium, $C_1$-$C_6$ alkyl, $C_1$-$C_6$ haloalkyl, 3- to 14-membered cycloalkyl, 3- to 14-membered cycloalkyl-$C_1$-$C_6$ alkyl, $C_6$-$C_{14}$ aryl, 5- to 14-membered heteroaryl, and 3- to 8-membered heterocyclyl.

According to the embodiments of the present disclosure, n is selected from 0, 1, and 2.

According to the embodiments of the present disclosure, $R^1$ is selected from halogen, cyano, and trifluoromethyl.

According to the embodiments of the present disclosure, $R^1$ is trifluoromethyl.

According to the embodiments of the present disclosure, $A^1$ is selected from

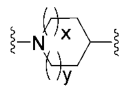

and $-N(R^3)-$, wherein x and y are each independently selected from 0, 1, and 2; $R^3$ is selected from H and $C_1$-$C_6$ alkyl.

According to the embodiments of the present disclosure, $A^1$ is selected from -NH-, $-N(CH_3)-$,

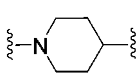, 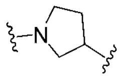 and 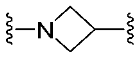.

According to the embodiments of the present disclosure, $A^1$ is selected from -NH-.

According to the embodiments of the present disclosure, $A^2$ is absent or selected from vinylene, ethynylene,

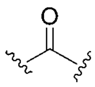, 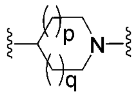, 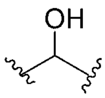, 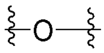, 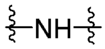, 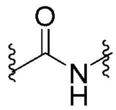,

$C_6$-$C_{10}$ arylene, and 5- to 10-membered heteroarylene (preferably, 5- to 10-membered heteroarylene containing one nitrogen atom), wherein p and q are each independently selected from 0, 1, and 2.

According to the embodiments of the present disclosure, $A^2$ is absent or selected from vinylene, ethynylene, phenylene,

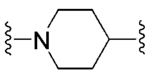, 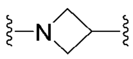, 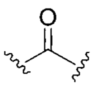, 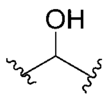, 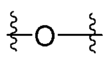, 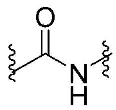,

and

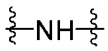.

According to the embodiments of the present disclosure, $A^2$ is selected from ethynylene,

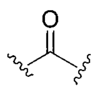,

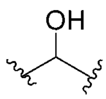, 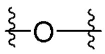, 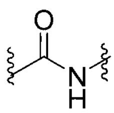, and 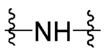.

According to the embodiments of the present disclosure,

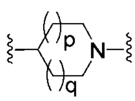

is unsubstituted or optionally substituted with one, two, or more (preferably, one) $R_{a2}$, and the $R_{a2}$ groups are identical or different, and are each independently selected from $C_1$-$C_6$ alkyl, $C_1$-$C_6$ alkyloxy, trifluoromethyl, 2,2-difluoroethyl,

methoxy, hydroxy, amino, methylamino, dimethylamino, acetylamino, carboxyl, methoxycarbonyl, methanesulfonyl, and nitro.

According to the embodiments of the present disclosure, $R^2$ is selected from the following groups unsubstituted or optionally substituted with one, two, or more (preferably, one) $R^{21}$: $C_6$-$C_{10}$ aryl, 5- to 10-membered heteroaryl (preferably, 5- to 10-membered heteroaryl containing one or two nitrogen or sulfur atoms), and 3- to 8-membered heterocyclyl (preferably, 3- to 8-membered heterocyclyl containing one nitrogen atom); the $R^{21}$ groups are identical or different and are each independently selected from hydrogen, halogen, $C_1$-$C_6$ alkyl, $C_1$-$C_6$ haloalkyl, $C_1$-$C_6$ alkyloxy, $C_1$-$C_6$ haloalkyloxy, 3- to 8-membered cycloalkyloxy, 3- to 8-membered cycloalkyl-$C_1$-$C_6$ alkyloxy, and $C_6$-$C_{10}$ aryl-C(O)-.

According to the embodiments of the present disclosure, $R^2$ is selected from substituted $C_6$-$C_{14}$ aryl and substituted 5- to 14-membered heteroaryl (preferably, 5- to 14-membered heteroaryl containing one or two nitrogen or sulfur atoms); the $C_6$-$C_{14}$ aryl or 5- to 14-membered heteroaryl is a monocyclyl or fused rings; the substituent is selected from hydrogen, halogen, cyano, trifluoromethyl, 2,2-difluoroethyl, $C_1$-$C_6$ alkyl, hydroxy, $C_1$-$C_6$ alkyloxy, amino, methylamino, ethylamino, dimethylamino, diethylamino, $C_3$-$C_6$ azacycloalkyl (preferably $C_3$-$C_6$ azacycloalkyl containing one nitrogen atom), formyl, acetyl, acetylamino, carbamoyl, and sulfamoyl; the substituent is monosubstituted or disubstituted.

According to the embodiments of the present disclosure, $R^2$ is selected from substituted $C_6$-$C_{10}$ aryl and substituted 5- to 10-membered heteroaryl (preferably, 5- to 10-membered heteroaryl containing one or two nitrogen or sulfur atoms), wherein the $C_6$-$C_{10}$ aryl is phenyl, naphthyl, anthryl, indanyl, indenyl, or 1,2,3,4-tetrahydronaphthyl, and the 5- to 10-membered heteroaryl is selected from pyridinyl, thienyl, pyrrolyl, furanyl, imidazolyl, pyrazolyl, pyrimidinyl, pyrazinyl, pyridazinyl, indolyl, indolinyl, quinolyl, isoquinolyl, tetrahydroisoquinolyl, indazolyl, benzothienyl, benzofuranyl, and benzimidazolyl; the substituent is selected from hydrogen, halogen, cyano, trifluoromethyl, 2,2-difluoroethyl, methyl, ethyl, hydroxy, methoxy, ethoxy, methylamino, ethylamino, dimethylamino, diethylamino, azetidinyl, tetrahydropyrrol-1-yl, piperidin-1-yl, morpholinyl, piperazinyl, N-methylpiperazinyl, acetyl, acetylamino, carbamoyl, and sulfamoyl; the substituent is monosubstituted or disubstituted.

According to the embodiments of the present disclosure, $R^2$ is selected from

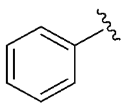, 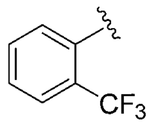,

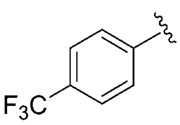, 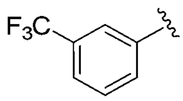, 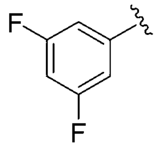, 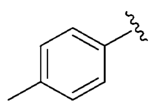, 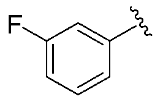, 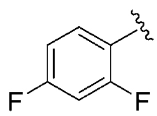,

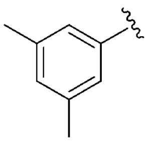, 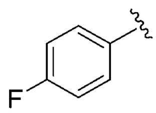, 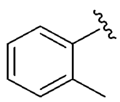, 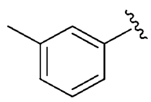, 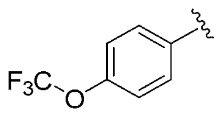, 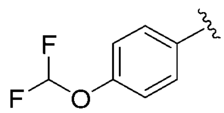,

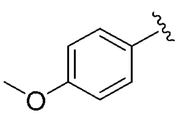, 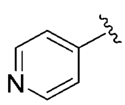, 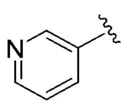, 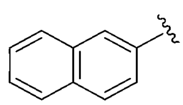, 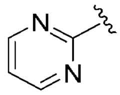, 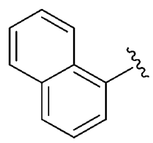, 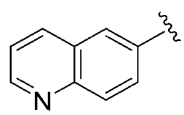,

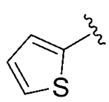, 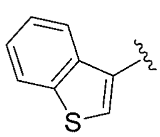, 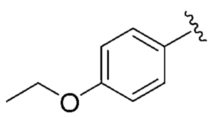, 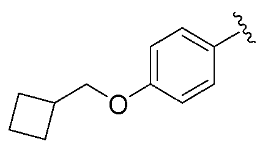, 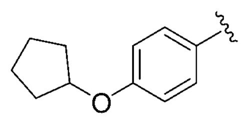,

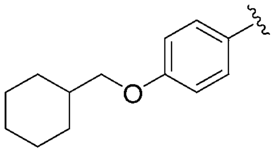, 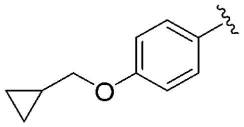, 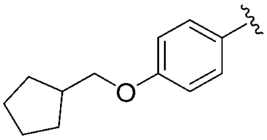, 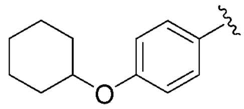,

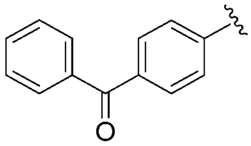, 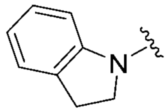, 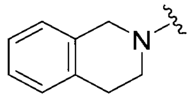, and 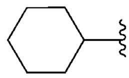.

According to the embodiments of the present disclosure, the compound of formula (I) is selected from the following compounds:

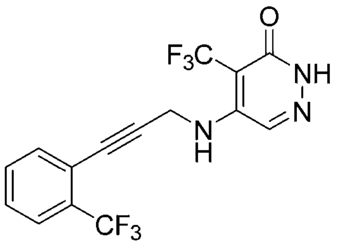

I-A-1

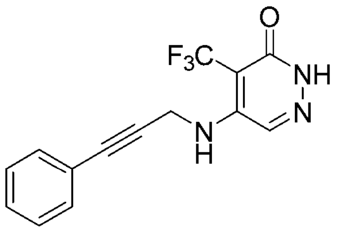

I-A-2

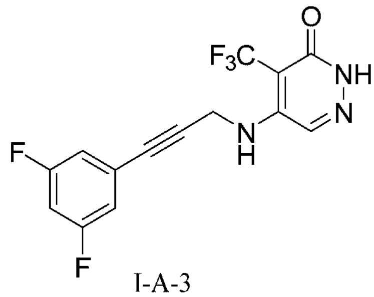

I-A-3

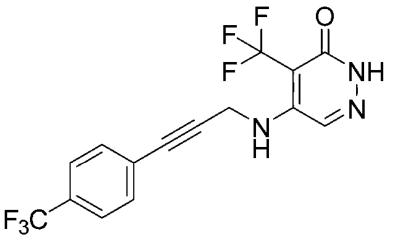

I-A-4

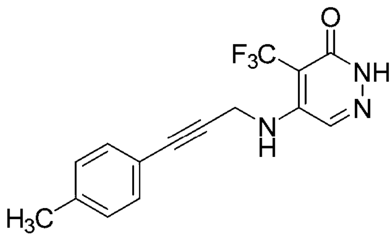

I-A-5

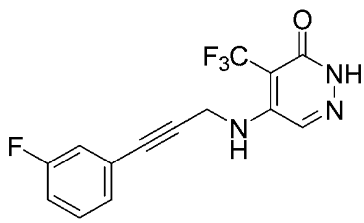

I-A-6

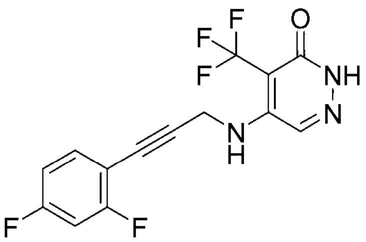

I-A-7

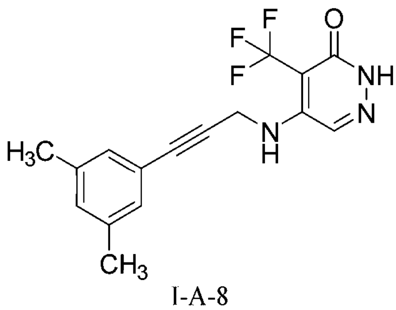

I-A-8

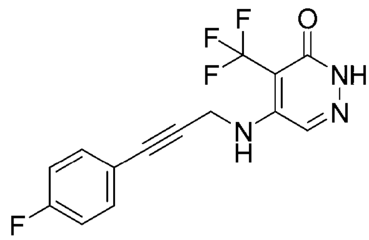

I-A-9

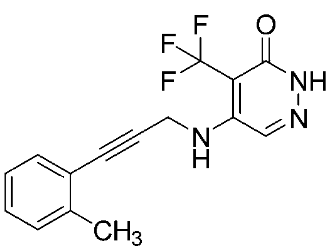

I-A-10

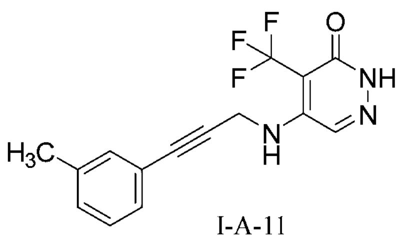

I-A-11

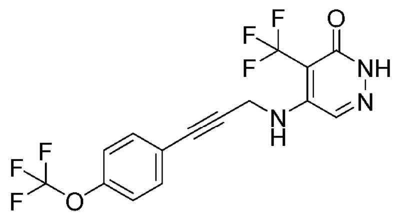

I-A-12

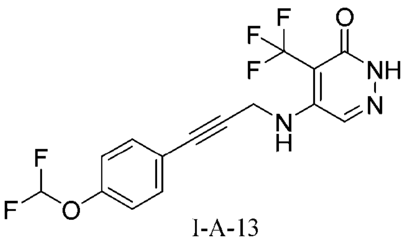
I-A-13

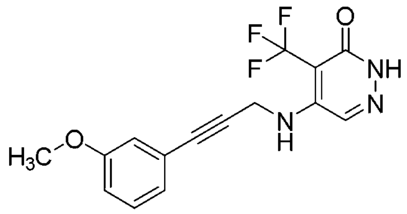
I-A-14

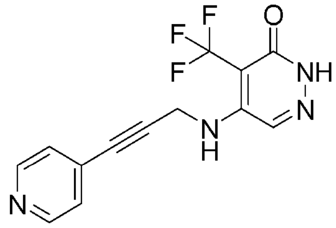
I-A-15

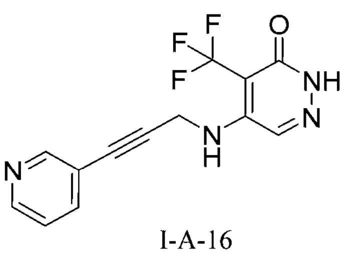
I-A-16

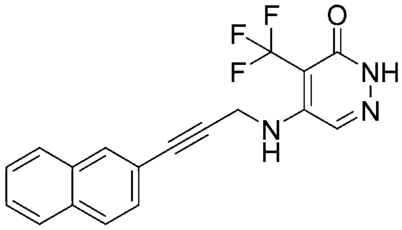
I-A-17

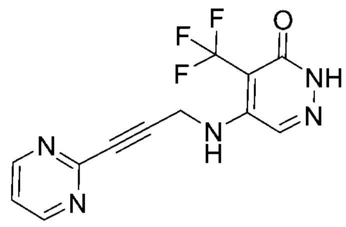
I-A-18

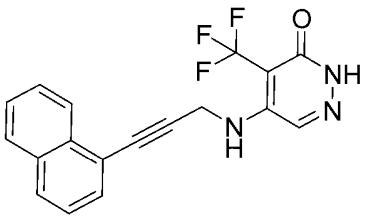
I-A-19

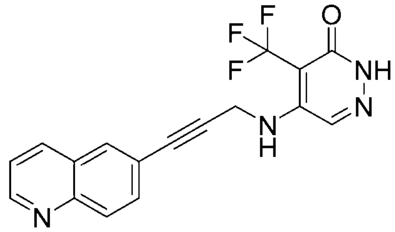
I-A-20

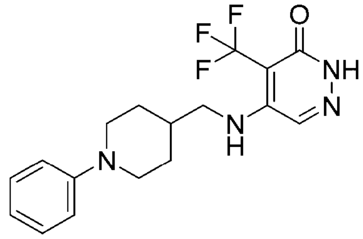
I-B-1

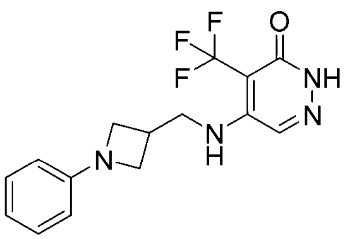
I-B-2

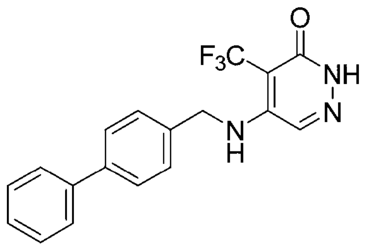
I-C-1

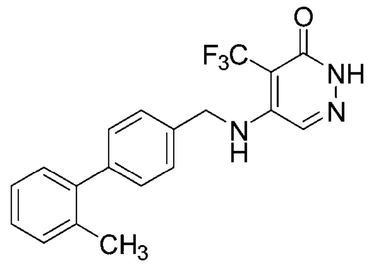
I-C-2

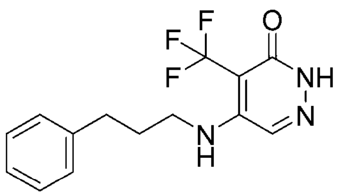
I-C-3

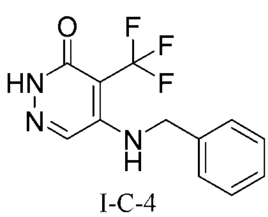
I-C-4

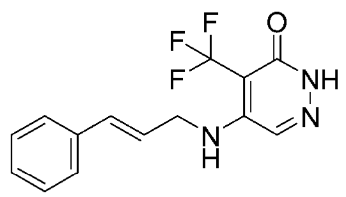
I-C-5

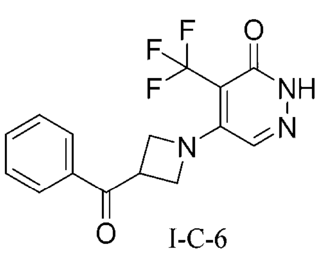
I-C-6

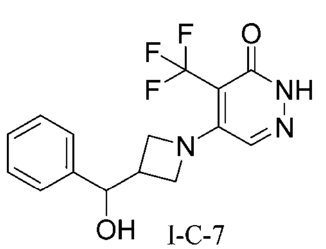
I-C-7

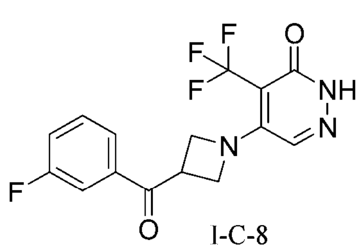
I-C-8

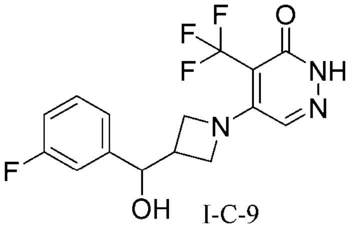
I-C-9

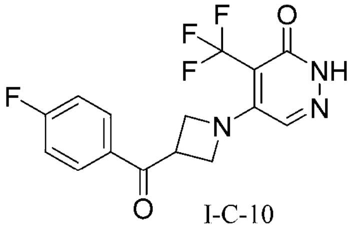
I-C-10

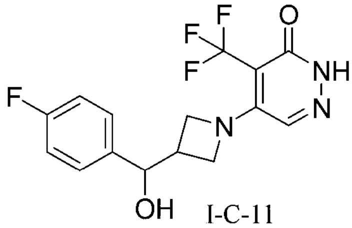
I-C-11

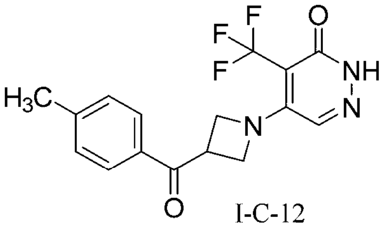
I-C-12

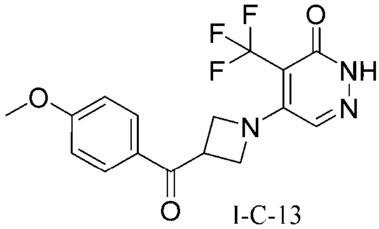
I-C-13

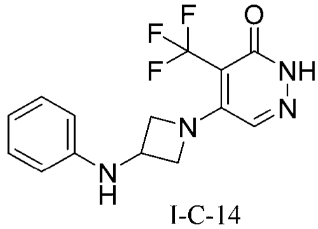
I-C-14

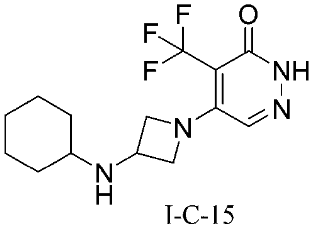
I-C-15

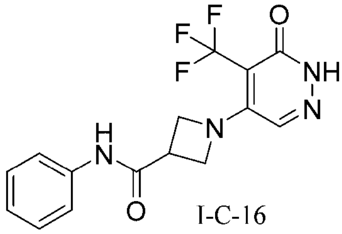
I-C-16

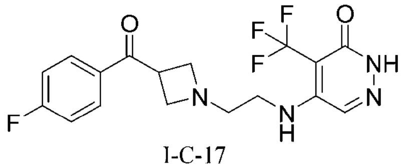
I-C-17

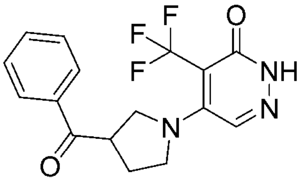
I-C-18

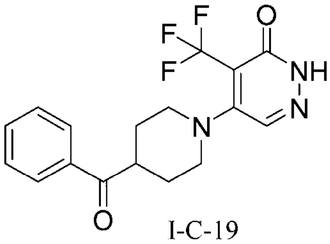
I-C-19

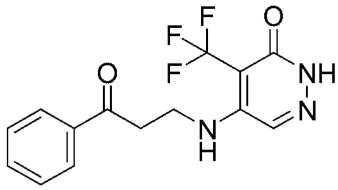
I-D-1

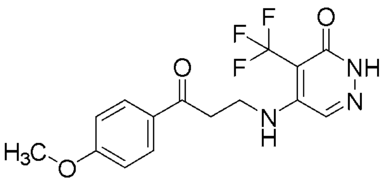
I-D-2

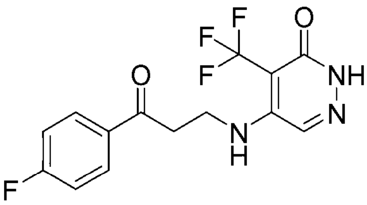
I-D-3

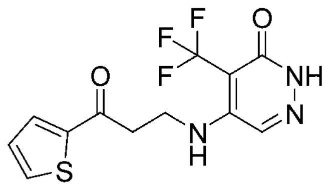
I-D-4

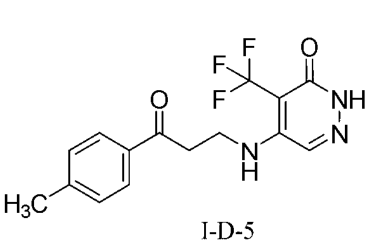
I-D-5

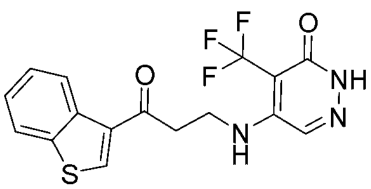
I-D-6

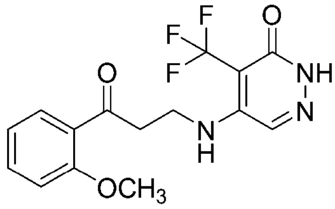
I-D-7

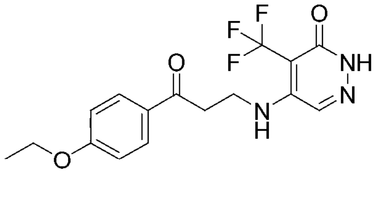

I-D-8

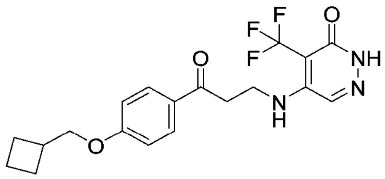

I-D-9

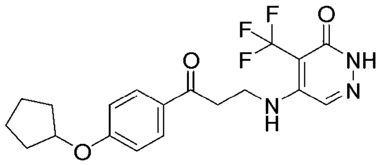

I-D-10

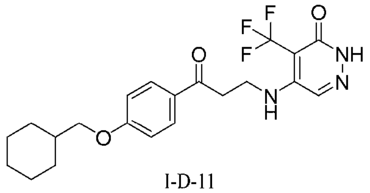

I-D-11

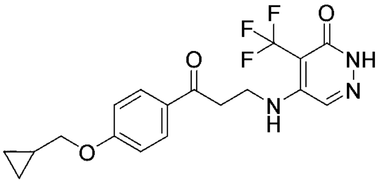

I-D-12

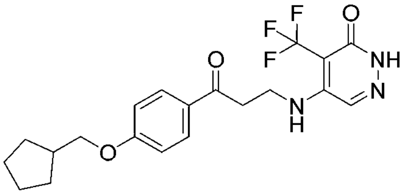

I-D-13

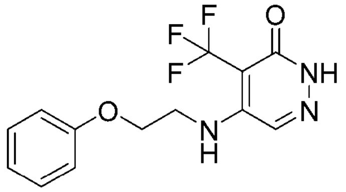

I-D-14

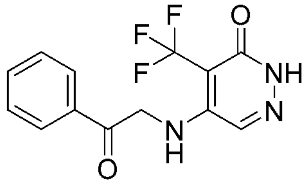

I-D-15

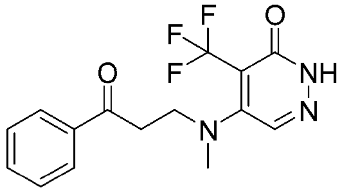

I-D-16

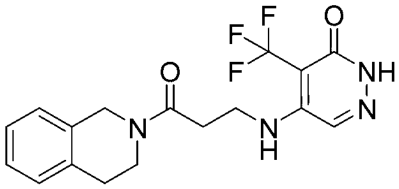

I-D-17

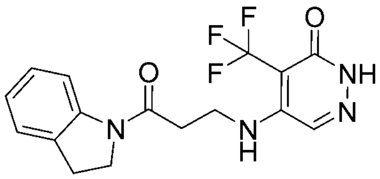

I-D-18 .

According to the embodiments of the present disclosure, the pharmaceutically acceptable salt is a salt formed by the compound and an acid, and the acid is selected from at least one of hydrochloric acid, hydrobromic acid, sulfuric acid, phosphoric acid, carbonic acid, methanesulfonic acid, benzenesulfonic acid, p-toluenesulfonic acid, naphthalenesulfonic acid, citric acid, malic acid, tartaric acid, lactic acid, pyruvic acid, acetic acid, maleic acid, succinic acid, fumaric acid, salicylic acid, phenylacetic acid, mandelic acid, and ferulic acid.

According to the embodiments of the present disclosure, the isotopic label is preferably a substitution of deuterium (D or $^2H$) for hydrogen ($^1H$).

The present disclosure further provides a method for preparing the compound, or the racemate, stereoisomer, tautomer, isotopically labeled form, solvate or pharmaceutically acceptable salt thereof, comprising:

(1) reacting compound I-1 with compound I-2 to give compound I-3; and
(2) removing PG from compound I-3 to give the compound of formula (I);

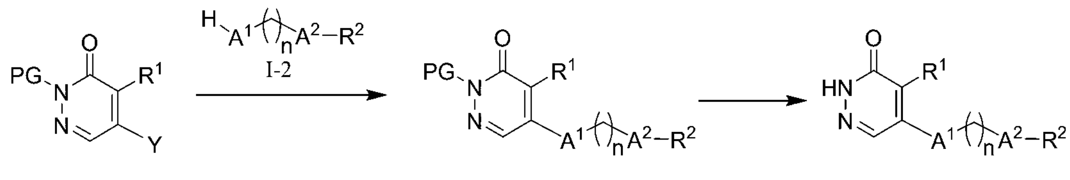


I-1 I-3 I

wherein Y is selected from a leaving group, for example, halogen, such as Cl and Br; PG is selected from a protecting

group, e.g., (trimethylsilyl)ethoxymethyl (SEM) and p-methoxybenzyl (PMB).

According to the embodiments of the present disclosure, the method further comprises at least one of the following schemes:
Scheme I: when $R^1$ is selected from trifluoromethyl, cyano, and halogen, $A^1$ denotes -NH- or -$N(CH_3)$-, $A^2$ is ethynylene, and n = 1, the compound of formula (I) has a structure of formula (I-A), and the method comprises:

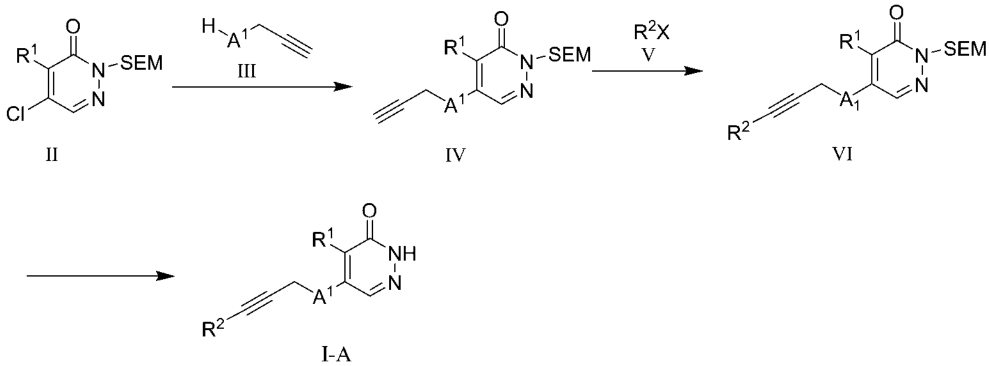


wherein the groups have the definitions as described herein, X is Cl, Br, or I, and SEM is (trimethylsilyl)ethoxymethyl.

(1-1) reacting compound II with compound III to give compound IV

According to the embodiments of the present disclosure, the reaction may be performed in the presence of a solvent, wherein the solvent is ethanol, N,N-dimethylformamide (DMF), N,N-dimethylacetamide, tetrahydrofuran (THF), 1,4-dioxane, glycol dimethyl ether, or acetonitrile, preferably 1,4-dioxane or ethanol.
According to the embodiments of the present disclosure, the reaction may be performed in the presence of an acid-binding agent, wherein the acid-binding agent is sodium carbonate, potassium carbonate, triethylamine, or N,Ndiisopropylethylamine (DIPEA), preferably DIPEA or triethylamine.

(1-2) reacting compound IV with compound V to give compound VI

According to the embodiments of the present disclosure, the reaction may be performed in the presence of a solvent, wherein the solvent is DMF, N,N-dimethylacetamide, THF, 1,4-dioxane, glycol dimethyl ether, or acetonitrile, preferably THF.
According to the embodiments of the present disclosure, the reaction may be performed in the presence of an acid-binding agent, wherein the acid-binding agent is sodium carbonate, potassium carbonate, triethylamine, or DIPEA, preferably triethylamine.
According to the embodiments of the present disclosure, the reaction may be performed in the presence of a catalyst, wherein the catalyst may be selected from a copper-based catalyst and a palladium-based catalyst; the copper-based catalyst is cuprous iodide or cuprous bromide, preferably cuprous iodide; the palladium-based catalyst is palladium acetate, bis(triphenylphosphine)palladium dichloride, tetrakis(triphenylphosphine)palladium(0), or 1,1'-bis(diphenylphosphino)ferrocenepalladium dichloride, preferably bis(triphenylphosphine)palladium dichloride.

(1-3) removing a protecting group from compound VI and performing an SEM reaction to give compound I-A

According to the embodiments of the present disclosure, the reaction may be performed in the presence of a solvent, wherein the solvent is THF, acetonitrile, or dichloromethane, preferably dichloromethane. According to the embodiments of the present disclosure, the reaction may be performed in the presence of an acid, wherein the acid is hydrochloric acid, trifluoroacetic acid, or trifluoromethanesulfonic acid, preferably trifluoroacetic acid or trifluoromethanesulfonic acid.
Scheme II: when $R^1$ is selected from trifluoromethyl, cyano, and halogen, $A^1$ denotes -NH-,

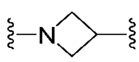,

or -$N(CH_3)$-, n = 1 or 0, $A^2$ is -NH- or

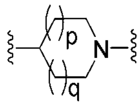

,

and p and q are each independently selected from 0, 1, and 2, the compound of formula (I) has a structure of formula (I-B), and the method comprises:

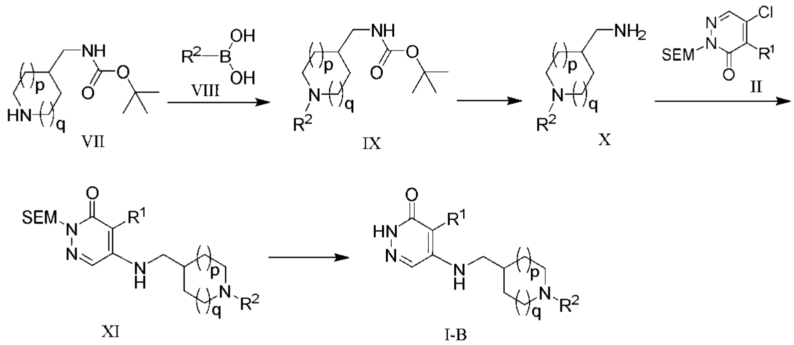


VII IX X XI I-B

wherein the groups have the definitions as described herein, and SEM is (trimethylsilyl)ethoxymethyl.

(2-1) reacting compound VII with compound VIII to give compound IX

According to the embodiments of the present disclosure, the reaction may be performed in the presence of a solvent, wherein the solvent is dichloromethane, toluene, DMF, N,N-dimethylacetamide, THF, 1,4-dioxane, glycol dimethyl ether, or acetonitrile, preferably dichloromethane.
According to the embodiments of the present disclosure, the reaction may be performed in the presence of an acid-binding agent, wherein the acid-binding agent is sodium carbonate, potassium carbonate, triethylamine, or DIPEA, preferably triethylamine.
According to the embodiments of the present disclosure, the reaction may be performed in the presence of a catalyst, wherein the catalyst may be selected from a copper-based catalyst, such as copper acetate.

(2-2) subjecting compound IX to a tert-butoxycarbonyl removal reaction to give compound X

According to the embodiments of the present disclosure, the reaction may be performed in the presence of a solvent, wherein the solvent is THF, acetonitrile, or dichloromethane, preferably dichloromethane.
According to the embodiments of the present disclosure, the reaction may be performed in the presence of an acid, wherein the acid is hydrochloric acid, trifluoroacetic acid, or trifluoromethanesulfonic acid, preferably trifluoroacetic acid.

(2-3) reacting compound X with compound II to give compound XI

According to the embodiments of the present disclosure, the reaction may be performed in the presence of a solvent, wherein the solvent is ethanol, DMF, N,N-dimethylacetamide, THF, 1,4-dioxane, glycol dimethyl ether, or acetonitrile, preferably 1,4-dioxane or ethanol.
According to the embodiments of the present disclosure, the reaction may be performed in the presence of an acid-binding agent, wherein the acid-binding agent is sodium carbonate, potassium carbonate, triethylamine, or DIPEA, preferably DIPEA or triethylamine.

(2-4) removing a protecting group from compound XI and performing an SEM reaction to give compound I-B

According to the embodiments of the present disclosure, the reaction may be performed in the presence of a solvent, wherein the solvent is THF, acetonitrile, or dichloromethane, preferably dichloromethane. According to the embodiments of the present disclosure, the reaction may be performed in the presence of an acid, wherein the acid is hydrochloric acid, trifluoroacetic acid, or trifluoromethanesulfonic acid, preferably trifluoroacetic acid.
Scheme III: when $R^1$ is selected from trifluoromethyl, cyano, and halogen, $A^1$ denotes -NH-, $-N(CH_3)-$,

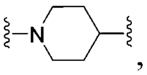, 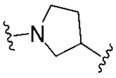, or 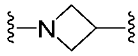,

$A^2$ is -CH=CH-, a benzene ring, $-CH_2-$,

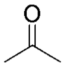, 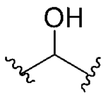, 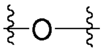, 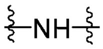, or 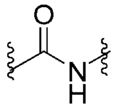, ;

and n is 0, 1, or 2, the compound of formula (I) has a structure of formula (I-C), and the method comprises:

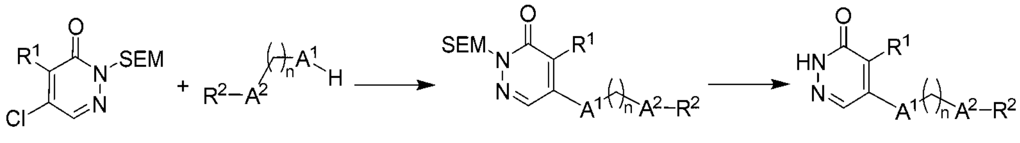

II XII XIII I-C

wherein the groups have the definitions as described herein, and SEM is (trimethylsilyl)ethoxymethyl.

(3-1) reacting compound II with compound XII to give compound XIII

According to the embodiments of the present disclosure, the reaction may be performed in the presence of a solvent, wherein the solvent is ethanol, DMF, N,N-dimethylacetamide, THF, 1,4-dioxane, glycol dimethyl ether, or acetonitrile, preferably 1,4-dioxane or ethanol.

According to the embodiments of the present disclosure, the reaction may be performed in the presence of an acid-binding agent, wherein the acid-binding agent is sodium carbonate, potassium carbonate, triethylamine, or DIPEA, preferably DIPEA or triethylamine.

(3-2) removing a protecting group from compound XIII and performing an SEM reaction to give compound I-C

According to the embodiments of the present disclosure, the reaction may be performed in the presence of a solvent, wherein the solvent is THF, acetonitrile, or dichloromethane, preferably dichloromethane. According to the embodiments of the present disclosure, the reaction may be performed in the presence of an acid, wherein the acid is hydrochloric acid, trifluoroacetic acid, or trifluoromethanesulfonic acid, preferably trifluoroacetic acid.

Scheme IV: when $R^1$ is selected from trifluoromethyl, cyano, and halogen, $A^1$ denotes -NH- or - $N(CH_3)$-, $A^2$ is

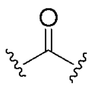,

and n is 1 or 2, the compound of formula (I) has a structure of formula (I-D), and the method comprises:

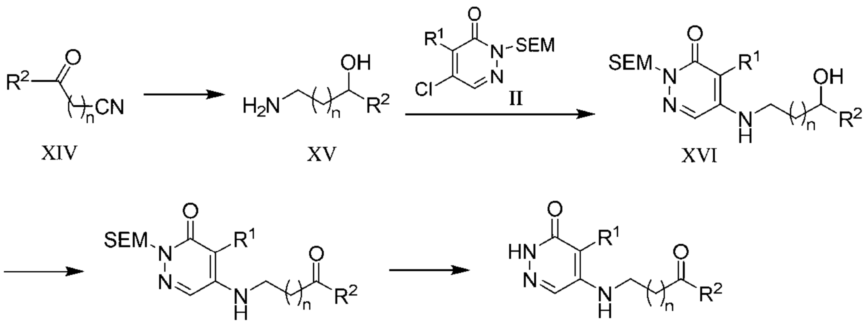

XIV XV II XVI XVII I-D-1

wherein the groups have the definitions as described herein, and SEM is (trimethylsilyl)ethoxymethyl.

(4-1) subjecting compound XIV to a reduction reaction in a hydrogen atmosphere to give compound XV

According to the embodiments of the present disclosure, the reaction may be performed in the presence of a solvent, wherein the solvent is THF, ethanol, or methanol, preferably methanol.
According to the embodiments of the present disclosure, the reaction may be performed in the presence of a catalyst, wherein the catalyst is palladium on carbon or palladium hydroxide, preferably palladium on carbon.

(4-2) reacting compound XV with compound II to give compound XVI

According to the embodiments of the present disclosure, the reaction may be performed in the presence of a solvent, wherein the solvent is ethanol, DMF, N,N-dimethylacetamide, THF, 1,4-dioxane, glycol dimethyl ether, or acetonitrile, preferably 1,4-dioxane or ethanol.
According to the embodiments of the present disclosure, the reaction may be performed in the presence of an acid-binding agent, wherein the acid-binding agent is sodium carbonate, potassium carbonate, triethylamine, or DIPEA, preferably DIPEA or triethylamine.

(4-3) subjecting compound XVI to an oxidation reaction to give compound XVII

According to the embodiments of the present disclosure, the reaction may be performed in the presence of a solvent, wherein the solvent is THF or dichloromethane, preferably dichloromethane.
According to the embodiments of the present disclosure, the reaction may be performed in the presence of an oxidant, wherein the oxidant is Dess-Martin periodinane, hydrogen peroxide, or pyridinium chlorochromate (PCC), preferably PCC.

(4-4) removing a protecting group from compound XVII and performing an SEM reaction to give compound I-D

According to the embodiments of the present disclosure, the reaction may be performed in the presence of a solvent, wherein the solvent is THF, acetonitrile, or dichloromethane, preferably dichloromethane. According to the embodiments of the present disclosure, the reaction may be performed in the presence of an acid, wherein the acid is hydrochloric acid, trifluoroacetic acid, or trifluoromethanesulfonic acid, preferably trifluoroacetic acid or trifluoromethanesulfonic acid.
According to the embodiments of the present disclosure, a corresponding acid is salified with the compound of formula (I) to give the pharmaceutically acceptable salt of the compound of formula (I). The present disclosure further provides a pharmaceutical composition, comprising a therapeutically effective amount of at least one of the compound of formula (I), or the racemate, stereoisomer, tautomer, isotopically labeled form, solvate or pharmaceutically acceptable salt thereof, and one or more pharmaceutically acceptable excipients.
According to the embodiments of the present disclosure, a pharmaceutically acceptable carrier may be added to the pharmaceutical composition to prepare a common pharmaceutical formulation, such as a tablet, a capsule, a syrup, a suspension, or an injection, or a common pharmaceutically acceptable excipient, such as a flavorant, a sweetener, a liquid/solid filler, or a diluent, may be added to the formulation.
The present disclosure further provides use of the compound of formula (I), or the racemate, stereoisomer, tautomer, isotopically labeled form, solvate or pharmaceutically acceptable salt thereof, or the pharmaceutical composition in preparing a medicament for preventing and/or treating a PARP-mediated disease, for example, in preparing a PARP inhibitor medicament.
According to the embodiments of the present disclosure, the subtype of PARP may be selected from PARP2, PARP3, PARP4 (VPARP), PARP5a (tankyrase1), PARP5b (tankyrase2), PARP7 (TiPARP), and/or sPARP1, preferably PARP7.
According to the embodiments of the present disclosure, the PARP-mediated disease may be a central nervous system disease, a cancer, an infection, an immune disease, a cardiovascular disease, or a metabolic disease; the central nervous system disease is, for example, stroke, such as ischemic stroke; the cancer is selected from lung cancer, pancreatic cancer, colorectal cancer, leukemia, Ewing's sarcoma, breast cancer, prostate cancer, T-cell lymphoma, B-cell lymphoma, malignant rhabdomyoma, synovial sarcoma, endometrioma, gastric cancer, liver cancer, renal cancer, melanoma, ovarian cancer, cerebral glioma, cholangiocarcinoma, nasopharyngeal cancer, cervical cancer, head and neck cancer, esophageal cancer, thyroid cancer, and bladder cancer.
The present disclosure further provides use of the compound of formula (I), or the racemate, stereoisomer, tautomer, isotopically labeled form, solvate or pharmaceutically acceptable salt thereof, or the pharmaceutical composition in preparing a medicament for preventing and/or treating a central nervous system disease, a cerebral ischemia-associated disease, a cancer, an infection, an immune disease, a cardiovascular disease, or a metabolic disease.

According to the embodiments of the present disclosure, the central nervous system disease is, for example, stroke, such as ischemic stroke; the cerebral ischemia-associated disease may be transient ischemic attack, cerebral thrombosis, or cerebral embolism; the cancer is selected from lung cancer, pancreatic cancer, colorectal cancer, leukemia, Ewing's sarcoma, breast cancer, prostate cancer, T-cell lymphoma, B-cell lymphoma, malignant rhabdomyoma, synovial sarcoma, endometrioma, gastric cancer, liver cancer, renal cancer, melanoma, ovarian cancer, cerebral glioma, cholangiocarcinoma, nasopharyngeal cancer, cervical cancer, head and neck cancer, esophageal cancer, thyroid cancer, and bladder cancer.

The present disclosure further provides a method for preventing and/or treating a PARP-mediated disease, comprising administering to a patient a prophylactically or therapeutically effective amount of at least one of the compound of formula (I), or the racemate, stereoisomer, tautomer, isotopically labeled form, solvate or pharmaceutically acceptable salt thereof, or a prophylactically or therapeutically effective amount of the pharmaceutical composition described above.

The present disclosure further provides at least one of the compound of formula (I), or the racemate, stereoisomer, tautomer, isotopically labeled form, solvate or pharmaceutically acceptable salt thereof, or the pharmaceutical composition thereof, for use in preventing and/or treating a PARP-mediated disease.

According to the embodiments of the present disclosure, the disease may be a central nervous system disease, a cancer, an infection, an immune disease, a cardiovascular disease, or a metabolic disease; the central nervous system disease is, for example, stroke, such as ischemic stroke; the cancer is selected from lung cancer, pancreatic cancer, colorectal cancer, leukemia, Ewing's sarcoma, breast cancer, prostate cancer, T-cell lymphoma, B-cell lymphoma, malignant rhabdomyoma, synovial sarcoma, endometrioma, gastric cancer, liver cancer, renal cancer, melanoma, ovarian cancer, cerebral glioma, cholangiocarcinoma, nasopharyngeal cancer, cervical cancer, head and neck cancer, esophageal cancer, thyroid cancer, and bladder cancer.

The present disclosure further provides a method for preventing and/or treating a cerebral ischemia-associated disease, comprising administering to a patient a prophylactically or therapeutically effective amount of at least one of the compound of formula (I), or the racemate, stereoisomer, tautomer, isotopically labeled form, solvate or pharmaceutically acceptable salt thereof, or a prophylactically or therapeutically effective amount of the pharmaceutical composition described above.

The present disclosure further provides at least one of the compound of formula (I), or the racemate, stereoisomer, tautomer, isotopically labeled form, solvate or pharmaceutically acceptable salt thereof, or the pharmaceutical composition thereof, for use in preventing and/or treating a cerebral ischemia-associated disease.

According to the embodiments of the present disclosure, the cerebral ischemia-associated disease may be transient ischemic attack, cerebral thrombosis, or cerebral embolism.

Beneficial Effects: The present disclosure has the following remarkable advantages compared with the prior art:

(1) The compound described herein has good membrane infiltration capacity, and the physicochemical parameters acquired by calculation meet the Clark-Lobell rules (the physicochemical property principles of a drug penetrating the blood-brain barrier: the total number of nitrogen atoms and oxygen atoms (N+O) is less than 6, the polar surface area (PSA) is less than 60-70, and the molecular weight is less than 450);
(2) The compound and the pharmaceutical composition thereof described herein also demonstrate an anti-tumor effect, good efficacy at the molecular level, and a nanomolar inhibitory level against the PARP7 enzyme;
(3) The method for preparing the compound features ease to operate.

## Definitions and Description

Unless otherwise stated, the terms used in the specification and claims have the following meanings. The term "alkyl" refers to a saturated aliphatic hydrocarbon group, which is a linear or branched group containing 1 to 20 carbon atoms, preferably an alkyl containing 1 to 12 (e.g., 1, 2, 3, 4, 5, 6, 7, 8, 9, 10, 11, and 12) carbon atoms, and more preferably an alkyl containing 1 to 6 carbon atoms ($C_{1-6}$ alkyl). Non-limiting examples of alkyl include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, various branched isomers thereof, and the like. The alkyl may be substituted or unsubstituted.

The term "alkyloxy" refers to -O-(alkyl), wherein the alkyl is as defined herein. Preferably, the term refers to an alkyloxy containing 1 to 12 (e.g., 1, 2, 3, 4, 5, 6, 7, 8, 9, 10, 11, and 12) carbon atoms ($C_{1-12}$ alkyloxy), and more preferably an alkyloxy containing 1 to 6 carbon atoms ($C_{1-6}$ alkyloxy). Non-limiting examples of alkyloxy include: methoxy, ethoxy, propoxy, and butoxy. The alkyloxy may be substituted or unsubstituted.

The term "alkylene" refers to a saturated divalent hydrocarbyl group obtained by removing two H from a saturated linear or branched hydrocarbyl, which may contain 1-12 carbon atoms. Non-limiting examples include methylene ($-CH_2-$), ethylene ($-CH_2CH_2-$), and the like. The alkylene may be substituted or unsubstituted.

The term "alkenyl" should be interpreted as preferably a linear or branched hydrocarbyl containing one or more double bonds and having 2-12 carbon atoms, preferably "$C_{2-10}$ alkenyl". The "$C_{2-10}$ alkenyl" should be interpreted as preferably a linear or branched monovalent hydrocarbyl group, which contains one or more double bonds and has 2, 3, 4, 5, 6, 7, 8, 9, or 10 carbon atoms, for example, 2, 3, 4, 5, or 6 carbon atoms (i.e., $C_{2-6}$ alkenyl) or 2 or 3 carbon atoms (i.e., $C_{2-3}$ alkenyl). It will be appreciated that when the alkenyl contains more than one double bond, the double bonds may be separated or conjugated. The alkenyl is, for example, vinyl, allyl, (E)-2-methylvinyl, (Z)-2-methylvinyl, (E)-but-2-enyl, (Z)-but-2-enyl, *(E)*-but-1-enyl, (Z)-but-1-enyl, pent-4-enyl, (E)-pent-3-enyl, (Z)-pent-3-enyl, (E)-pent-2-enyl, (Z)-pent-2-enyl, (E)-pent-1-enyl, *(Z)*-pent-1-enyl, hex-5-enyl, (E)-hex-4-enyl, (Z)-hex-4-enyl, (E)-hex-3-enyl, (Z)-hex-3-enyl, (E)-hex-2-enyl, (Z)-hex-2-enyl, (E)-hex-1-enyl, (Z)-hex-1-enyl, isopropenyl, 2-methylprop-2-enyl, 1-methylprop-2-enyl, 2-methylprop-1-enyl, (E)-1-methylprop-1-enyl, (Z)-1-methylprop-1-enyl, 3-methylbut-3-enyl, 2-methylbut-3-enyl, 1-methylbut-3-enyl, 3-methylbut-2-enyl, (E)-2-methylbut-2-enyl, (Z)-2-methylbut-2-enyl, *(E)*-1-methylbut-2-enyl, (Z)-1-methylbut-2-enyl, (E)-3-methylbut-1-enyl, (Z)-3-methylbut-1-enyl, *(E)*-2-methylbut-1-enyl, (Z)-2-methylbut-1-enyl, (E)-1-methylbut-1-enyl, (Z)-1-methylbut-1-enyl, 1,1-dimethylprop-2-enyl, 1-ethylprop-1-enyl, 1-propylvinyl, or 1-isopropylvinyl. The alkenyl may be substituted or unsubstituted.

The term "alkynyl" should be interpreted as a linear or branched monovalent hydrocarbyl containing one or more triple bonds and having 2-12 carbon atoms, preferably "$C_{2-10}$ alkynyl". The term "$C_{2-10}$ alkynyl" should be interpreted as preferably a linear or branched monovalent hydrocarbyl group, which contains one or more triple bonds and has 2, 3, 4, 5, 6, 7, 8, 9, or 10 carbon atoms, for example, 2, 3, 4, 5, or 6 carbon atoms (i.e., "$C_{2-6}$ alkynyl") or 2 or 3 carbon atoms ("$C_{2-3}$ alkynyl"). The alkynyl is, for example, ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl, or 3,3-dimethylbut-1-ynyl. In particular, the alkynyl is ethynyl, prop-1-ynyl, or prop-2-ynyl. The alkynyl may be substituted or unsubstituted.

The term "cycloalkyl" refers to a saturated or partially unsaturated monocyclic or polycyclic hydrocarbon substituent. The cycloalkyl ring contains 3 to 14 carbon atoms, preferably 3 to 8 (e.g., 3, 4, 5, 6, 7, and 8) carbon atoms, and more preferably 3 to 6 carbon atoms. The ring atoms may optionally be substituted with oxo, and the oxo (=O) on the ring belongs to a part of the ring. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. The polycyclic cycloalkyl includes spiro, fused, and bridged cycloalkyl.

The term "heterocyclyl" refers to a saturated or unsaturated non-aromatic ring or ring system having 3 to 14 members, which, for example, is a 3-, 4-, 5-, 6-, or 7-membered monocyclic ring system, a 7-, 8-, 9-, 10-, 11-, or 12-membered bicyclic (e.g., fused, bridged, or spiro) ring system, or a 10-, 11-, 12-, 13-, or 14-membered tricyclic ring system, and contains at least one, e.g., 1, 2, 3, 4, 5, or more, heteroatoms selected from O, S, and N, wherein N and S may also be optionally oxidized to various oxidized forms to form nitrogen oxides, -S(O)-, or $-S(O)_2-$. Unless otherwise stated, the heterocyclyl may be carbon- or nitrogen-based, and the $-CH_2-$ group may be optionally substituted with -C(=O)-. The sulfur atom of the ring may be optionally oxidized to a S-oxide. The nitrogen atom of the ring may be optionally oxidized to a N-oxide. In some embodiments, the heterocyclyl is a heterocyclyl consisting of 5 to 12 atoms. In some other embodiments, the heterocyclyl is a heterocyclyl consisting of 5 to 8 atoms. In still some other embodiments, the heterocyclyl is a heterocyclyl consisting of 5 to 7 atoms. In yet some other embodiments, the heterocyclyl is a heterocyclyl consisting of 5 to 6 atoms. The heterocyclyl may also be a bicyclic heterocyclyl; in some embodiments, the heterocyclyl is a bicyclic heterocyclyl consisting of 7-12 atoms; in some other embodiments, the heterocyclyl is a bicyclic heterocyclyl consisting of 7-10 atoms; in still other embodiments, the heterocyclyl is a bicyclic heterocyclyl consisting of 8-10 atoms.

The term "aryl" should be interpreted as preferably an aromatic or partially aromatic monovalent monocyclic, bicyclic, or tricyclic hydrocarbon ring having 6, 7, 8, 9, 10, 11, 12, 13, or 14 carbon atoms ("$C_{6-14}$ aryl"), in particular a ring having 6 carbon atoms ("$C_6$ aryl"), such as phenyl; or biphenyl, a ring having 9 carbon atoms ("$C_9$ aryl"), such as indanyl or indenyl, a ring having 10 carbon atoms ("$C_{10}$ aryl"), such as tetrahydronaphthyl, dihydronaphthyl or naphthyl, a ring having 13 carbon atoms ("$C_{13}$ aryl"), such as fluorenyl, or a ring having 14 carbon atoms ("$C_{14}$ aryl"), such as anthryl. When the $C_{6-20}$ aryl is substituted, it may be monosubstituted or polysubstituted. In addition, the substitution site is not limited, and may be, for example, ortho-substitution, para-substitution, or meta-substitution. The aryl group includes a ring system formed by fusing an aromatic ring and another aromatic ring, or an aromatic ring and a non-aromatic carbon ring. Examples of the aryl group may include phenyl, naphthyl, anthryl, 1,2,3,4-tetrahydronaphthyl, 2,3-dihydro-1*H*-indenyl, and bicyclo [4,2,0]octa-1(6),2,4-trienyl. The aryl group may be substituted or unsubstituted, wherein the substituents include, but are not limited to, fluorine, chlorine, bromine, oxo (=O), cyano, nitro, carboxyl, hydroxy, amino, aminomethyl, aminoacyl, methylamino, phenylamino, hydroxymethyl, methylsulfonyl, aminosulfonyl, acetyl, methoxy, phenoxy, trifluoromethoxy, methyl, ethyl, propyl, isopropyl, n-butyl, tert-butyl, cyclopropyl, cyclopentyl, cyclohexyl, tetrahydrofuranyl, tetrahydrothienyl, tetrahydropyrrolyl, imidazolyl, imidazolinyl, piperidinyl, piperazinyl, morpholinyl, thienyl, thiazolyl, furanyl, pyrrolyl, phenyl, pyridinyl, pyrimidinyl, $-C(=NH)NH_2$, trifluoromethyl, or the like.

The term "heteroaryl" should be interpreted as a monovalent monocyclic, bicyclic (e.g., fused, bridged or spiro) or tricyclic ring system containing 5 to 14 ring atoms. The heteroaryl has 5 to 14 ring atoms, including 1 to 5 heteroatoms independently selected from N, O, and S, wherein at least one ring (or the whole ring) is aromatic. The heteroaryl has one or more attachment points connected to the rest of the molecule. The term "heteroaryl" may be used interchangeably with the term "heteroaromatic ring" or "heteroaromatic compound". The heteroaryl includes a ring system formed by fusing a heteroaromatic ring and an aromatic ring, a heteroaromatic ring and another heteroaromatic ring, or a heteroaromatic ring and a non-aromatic carbon ring or heterocyclic ring. In some embodiments, the heteroaryl consisting of 5 to 10 atoms contains 1, 2, 3, or 4 heteroatoms independently selected from oxygen, sulfur, and nitrogen. In some embodiments, the heteroaryl is a heteroaryl consisting of 7 to 12 atoms, including 1, 2, 3, or 4 heteroatoms independently selected from O, S, and N; the heteroaryl consisting of 7 to 12 atoms may be a monocyclic ring system or a bicyclic ring system containing two rings. In some other embodiments, the heteroaryl is a heteroaryl consisting of 7 to 10 atoms, including 1, 2, 3, or 4 heteroatoms independently selected from oxygen, sulfur, and nitrogen; the heteroaryl consisting of 7 to 10 atoms may be a monocyclic ring system or a bicyclic ring system containing two rings.

The term "halogen" refers to F, Cl, Br, or I.

The term "hydroxy" refers to -OH.

The term "amino" refers to $-NH_2$.

The term "cyano" refers to -CN.

The term "nitro" refers to $-NO_2$.

The term "oxo" refers to "=O".

The term "carbonyl" refers to C=O.

The term "carboxyl" refers to -C(O)OH.

The term "patient" refers to any animal including mammals, preferably mice, rats, other rodents, rabbits, dogs, cats, pigs, cattle, sheep, horses, or primates, and most preferably humans.

The term "therapeutically effective amount" refers to the amount of the active compound or drug that induces a biological or medical response pursued by researchers, veterinarians, physicians, or other clinicians in tissues, systems, animals, individuals, or humans, including one or more of the following effects: (1) disease prevention: for example, the prevention of a disease, disorder, or condition in an individual who is susceptible to the disease, disorder, or condition but has not yet experienced or exhibited the pathology or symptoms of the disease; (2) disease inhibition: for example, the inhibition of a disease, disorder, or condition in an individual who is experiencing or exhibiting the pathology or symptoms of the disease, disorder, or condition (i.e., the prevention of the further progression of the pathology and/or symptoms); (3) disease alleviation: for example, the alleviation of a disease, disorder, or condition in an individual who is experiencing or exhibiting the pathology or symptoms of the disease, disorder, or condition (i.e., the reverse of the pathology and/or symptoms).

## DETAILED DESCRIPTION

The technical schemes of the present disclosure will be further illustrated in detail with reference to the following specific examples. It will be appreciated that the following examples are merely exemplary illustration and explanation of the present disclosure, and should not be construed as limiting the claimed scope of the present disclosure. All techniques implemented on the basis of the above content of the present disclosure are encompassed within the claimed scope of the present disclosure.

Unless otherwise stated, the starting materials and reagents used in the following examples are all commercially available products or can be prepared by known methods.

Example 1: Synthesis of 4-trifluoromethyl-5-((3-(2-(trifluoromethyl)phenyl)prop-2-yn-1-yl)amino)pyridazin-3(2 H)-one **(I-A-1)**

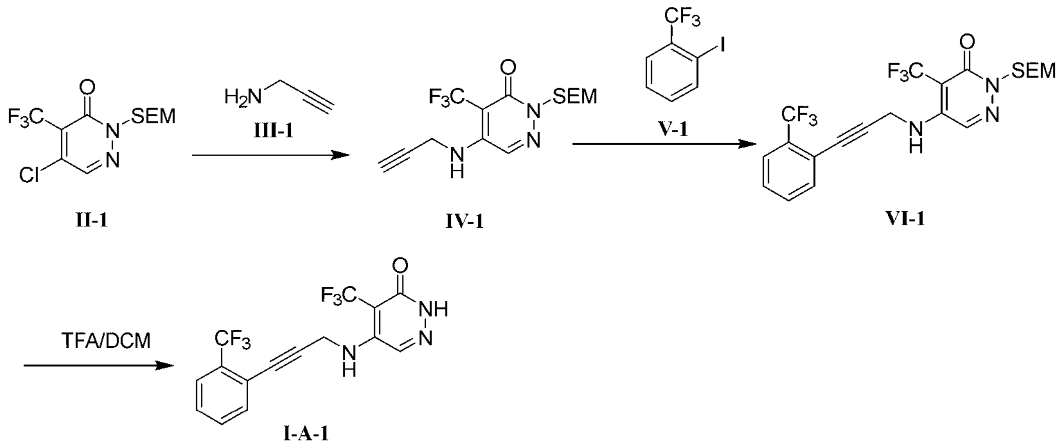

II-1
IV-1
VI-1
I-A-1

Synthesis of 5-(prop-2-yn-1-ylamino)-4-trifluoromethyl-2-((2-(trimethylsilyl)ethoxy)methyl) pyridazin-3(2 *H*)-one **(IV-1)**

The compound 5-chloro-4-trifluoromethyl-2-((2-(trimethylsilyl)ethoxy)methyl)pyridazin-3(2 *H*)-one **(II-1)** (7.0 g, 22.0 mmol) was dissolved in 20 mL of 1,4-dioxane, and propargylamine **(III-1)** (1.3 g, 24.2 mmol) and DIPEA (8.5 g, 66.0 mmol) were added. The mixture was heated to 70 °C and incubated for 0.5 h. The reaction was monitored by thin-layer chromatography (Vpetroleum ether: Vethyl acetate = 4:1) until completion. 15 mL of water was added, and the mixture was extracted with ethyl acetate (10 mL × 3). The organic phases were combined, washed with saturated brine, dried over anhydrous sodium sulfate, and filtered in vacuum, and the filtrate was concentrated. The crude product was purified by silica gel column chromatography (*V*petroleum ether: *V*ethyl acetate = 8:1). The crude product was purified to give a brown oily substance **(IV-1,** 4.1 g, 53.7% yield). ESI-MS $[M+H]^+$348.1. Synthesis of 4-trifluoromethyl-5-((3-(2-(trifluoromethyl) phenyl)prop-2-yn-1-yl)amino)-2-((2-(trimethylsilyl)ethoxy)methyl)pyridazin-3(2 *H*)-one **(VI-1)**

Compound **IV-1** (365.4 mg, 1.0 mmol) was dissolved in 3 mL of anhydrous tetrahydrofuran, and o-iodobenzotrifluoride **(V-1)** (299.2 mg, 1.1 mmol) was added, followed by the addition of cuprous iodide (38.1 mg, 0.2 mmol), bis(triphenylphosphine)palladium dichloride (70.2 mg, 0.1 mmol), and triethylamine (303.0 mg, 3.0 mmol). The mixture was purged with nitrogen, heated to 70 °C, and incubated for 0.5 h. The reaction was monitored by thin-layer chromatography (Vpetroleum ether: Vethyl acetate = 2:1) until completion. 10 mL of water was added, and the mixture was extracted with ethyl acetate (5 mL × 3). The organic phases were combined, washed with saturated brine, dried over anhydrous sodium sulfate, and filtered in vacuum, and the filtrate was concentrated. The crude product was purified by silica gel column chromatography (*V*petroleum ether: *Vethyl* acetate = 8:1). The crude product was purified to give a yellow solid **(VI-1,** 0.4 g, 81.5% yield). ESI-MS $[M+H]^+$492.1.

Synthesis of 4-trifluoromethyl-5-((3-(2-(trifluoromethyl)phenyl)prop-2-yn-1-yl)amino)pyridazin-3(2 *H*)-one **(I-A-1)**

Compound **VI-1** (245.5 mg, 0.5 mmol) was dissolved in 3 mL of dichloromethane before 3 mL of trifluoroacetic acid was added. The system was incubated at room temperature for 3 h. The reaction was monitored by thin-layer chromatography (Vdichloromethane: Vmethanol = 15:1) until completion. The reaction mixture was concentrated by rotary evaporation at reduced pressure to remove the solvent, and the mixture was adjusted to pH 8 by adding a saturated aqueous sodium bicarbonate solution. 10 mL of water was added, and the resultant mixture was extracted with dichloromethane (10 mL × 3). The organic phases were combined, washed with a saturated aqueous sodium chloride solution, dried over anhydrous sodium sulfate, and filtered in vacuum, and the filtrate was concentrated. The crude product was purified by silica gel column chromatography (*V*dichloromethane: *V*methanol = 50:1) to give a white solid **(I-A-1,** 124.0 mg, 68.9% yield). ESI-MS $[M+H]^+$362.1; $^1H$ NMR (400 MHz, DMSO-$d_6$) $\delta$ 12.64 (s, 1H), 7.94 (s, 1H), 7.77 (d, *J* = 7.8 Hz, 1H), 7.69-7.65 (m, 2H), 7.63-7.56 (m, 1H), 7.56-7.52 (m, 1H), 4.50 (d, *J* = 5.9 Hz, 2H).

The following compounds were prepared with reference to the method for preparing compound **I-A-1:**

| Compound No. | $^1H$-NMR | MS |
|---|---|---|
| Example 2 (I-A-2) | $^1H$ NMR (400 MHz, DMSO-$d_6$) $\delta$ 12.67 (s, 1H), 7.99 (s, 1H), 7.59-7.52 (m, 1H), 7.45-7.40 (m, 2H), 7.39-7.34 (m, 3H), 4.45 (d, *J* = 5.7 Hz, 2H). | ESI-MS: $[M+H]^+$ 294.1 |
| Example 3 (I-A-3) | $^1H$ NMR (400 MHz, DMSO-$d_6$) $\delta$ 12.68 (s, 1H), 7.96 (s, 1H), 7.61-7.49 (m, 1H), 7.38-7.30 (m, 1H), 7.24-7.17 (m, 2H), 4.47 (d, *J* = 5.7 Hz, 2H). | ESI-MS: $[M+H]^+$ 330.1 |

(continued)

| Compound No. | $^1$H-NMR | MS |
|---|---|---|
| Example 4 (I-A-4) | $^1$H NMR (300 MHz, DMSO-$d_6$) $\delta$ 12.70 (s, 1H,), 8.00 (s, 1H), 7.73 (d, $J$ = 8.2 Hz, 2H), 7.64 (d, $J$ = 8.2 Hz, 2H), 7.60-7.56 (m, 1H), 4.49 (d, $J$ = 5.8 Hz, 2H). | ESI-MS: $[M+H]^+$ 362.1 |
| Example 5 (I-A-5) | $^1$H NMR (300 MHz, DMSO-$d_6$) $\delta$ 12.66 (s, 1H), 7.98 (s, 1H), 7.60-7.50 (m, 1H), 7.30 (d, $J$ = 7.8 Hz, 2H), 7.17 (d, $J$ = 7.8 Hz, 2H), 4.43 (d, $J$ = 5.8 Hz, 2H), 2.30 (s, 3H). | ESI-MS: $[M+H]^+$ 308.1 |
| Example 6 (I-A-6) | $^1$H NMR (300 MHz, DMSO-$d_6$) $\delta$ 12.68 (s, 1H), 7.98 (s, 1H), 7.59-7.52 (m, 1H), 7.46-7.38 (m, 1H), 7.32-7.21 (m, 3H), 4.46 (d, $J$ = 5.7 Hz, 2H). | ESI-MS: $[M+H]^+$ 312.2 |
| Example 7 (I-A-7) | $^1$H NMR (300 MHz, DMSO-$d_6$) $\delta$ 12.68 (s, 1H), 7.97 (s, 1H), 7.62 -7.59 (m, 1H), 7.60-7.50 (m, 1H), 7.42-7.35 (m, 1H), 7.15-7.09 (m, 1H), 4.48 (d, $J$ = 5.8 Hz, 2H). | ESI-MS: $[M+H]^+$330.1 |
| Example 8 (I-A-8) | $^1$H NMR (300 MHz, DMSO-$d_6$) $\delta$ 12.66 (s, 1H), 7.96 (s, 1H), 7.58-7.52 (m, 1H), 7.02 (s, 3H), 4.43 (d, $J$ = 5.8 Hz, 2H), 2.23 (s, 6H). | ESI-MS: $[M+H]^+$ 322.1 |
| Example 9 (I-A-9) | $^1$H NMR (300 MHz, DMSO-$d_6$) $\delta$ 12.67 (s, 1H), 7.98 (s, 1H), 7.59-7.54 (m, 1H), 7.50-7.44 (m, 2H), 7.25-7.18 (m, 2H), 4.43 (d, $J$ = 5.8 Hz, 2H). | ESI-MS: $[M+H]^+$ 312.2 |
| Example 10 (I-A-10) | $^1$H NMR (300 MHz, DMSO-$d_6$) $\delta$ 12.64 (s, 1H), 8.02 (s, 1H), 7.59-7.54 (m, 1H), 7.37-7.34 (m, 1H), 7.28-7.26 (m, 2H), 7.20-7.14 (m, 1H), 4.49 (d, $J$ = 5.9 Hz, 2H), 2.30 (s, 3H). | ESI-MS: $[M+H]^+$ 308.1 |
| Example 11 (I-A-11) | $^1$H NMR (300 MHz, DMSO-$d_6$) $\delta$ 12.65 (s, 1H), 7.97 (s, 1H), 7.55-7.52 (m, 1H), 7.28-7.18 (m, 4H), 4.44 (d, $J$ = 5.8 Hz, 2H), 2.28 (s, 3H). | ESI-MS: $[M+H]^+$ 308.1 |
| Example 12 (I-A-12) | $^1$H NMR (300 MHz, DMSO-$d_6$) $\delta$ 12.68 (s, 1H), 7.98 (s, 1H), 7.59-7.57 (m, 1H), 7.56-7.54 (m, 2H), 7.39 (d, $J$ = 8.3 Hz, 2H), 4.47 (d, $J$ = 5.7 Hz, 2H). | ESI-MS: $[M+H]^+$ 378.1 |
| Example 13 (I-A-13) | $^1$H NMR (300 MHz, DMSO-$d_6$) $\delta$ 12.48 (s, 1H), 7.97 (s, 1H), 7.60-7.45 (m, 3H), 7.30-7.00 (m, 3H), 4.45-4.43 (m, 2H). | ESI-MS: $[M+H]^+$ 360.1 |
| Example 14 (I-A-14) | $^1$H NMR (300 MHz, DMSO-$d_6$) $\delta$ 12.65 (s, 1H), 7.98 (s, 1H), 7.55-7.51 (m, 1H), 7.28 (t, $J$ = 7.8 Hz, 1H), 7.00-6.94 (m, 3H), 4.44 (d, $J$ = 5.7 Hz, 2H), 3.75 (s, 3H). | ESI-MS: $[M+H]^+$ 324.1 |
| Example 15 (I-A-15) | $^1$H NMR (300 MHz, DMSO-$d_6$) $\delta$ 12.68 (s, 1H), 8.57 (s, 2H), 7.98 (s, 1H), 7.55 (s, 1H), 7.39 (s, 2H), 4.51 (s, 2H). | ESI-MS: $[M+H]^+$ 295.1 |
| Example 16 (I-A-16) | $^1$H NMR (300 MHz, DMSO-$d_6$) $\delta$ 12.68 (s, 1H), 8.62 (s, 1H), 8.56 (d, $J$ = 5.7 Hz, 1H), 7.99 (s, 1H), 7.86 (d, $J$ = 7.9 Hz, 1H), 7.56 (s, 1H),7.41 (t, $J$ = 6.5 Hz, 1H), 4.49 (d, $J$ = 5.8 Hz, 2H). | ESI-MS: $[M+H]^+$ 295.1 |
| Example 17 (I-A-17) | $^1$H NMR (300 Hz, DMSO-$d_6$) $\delta$ 12.69 (s, 1H), 8.05 (d, $J$ = 6.0 Hz, 2H), 7.95-7.88 (m, 3H), 7.63-7.52 (m, 3H), 7.47 (d, $J$ = 8.3 Hz, 1H), 4.51 (d, $J$ = 4.7 Hz, 2H). | ESI-MS: $[M+H]^+$ 344.1 |
| Example 18 (I-A-18) | $^1$H NMR (300 Hz, DMSO-$d_6$) $\delta$ 12.72 (s, 1H), 8.80 (d, $J$ = 4.9 Hz, 2H), 8.00 (s, 1H), 7.65-7.56 (m, 1H), 7.51 (t, $J$ = 5.0 Hz, 1H), 4.53 (d, $J$ = 5.6 Hz, 2H). | ESI-MS: $[M+H]^+$ 296.1 |
| Example 19 (I-A-19) | $^1$H NMR (300 Hz, DMSO-$d_6$) $\delta$ 12.69 (s, 1H), 8.20-8.17 (m, 1H), 8.16 (s, 1H), 7.99-7.96 (m, 2H), 7.68 (d, $J$ = 6.9 Hz, 2H), 7.61-7.58 (m, 2H), 7.50 (t, $J$ = 7.7 Hz, 1H), 4.62 (d, $J$ = 5.5 Hz, 2H). | ESI-MS: $[M+H]^+$ 344.1 |
| Example 20 (I-A-20) | $^1$H NMR (300 Hz, DMSO-$d_6$) $\delta$ 12.69 (s, 1H), 8.94-8.89 (m, 1H), 8.37 (d, $J$ = 7.9 Hz, 1H), 8.47-8.10 (m, 1H), 8.04 (s, 1H), 7.99 (d, $J$ = 8.7 Hz, 1H), 7.73-7.70 (m, 1H), 7.62-7.59 (m, 1H), 7.57-7.54 (m, 1H), 4.52 (d, $J$ = 5.7 Hz, 2H). | ESI-MS: $[M+H]^+$ 345.1 |

Example 21: Synthesis of 5-(((1-phenylpiperidin-4-yl)methyl)amino)-4-trifluoromethylpyridazin-3(2 *H*)-one **(I-B-1)**

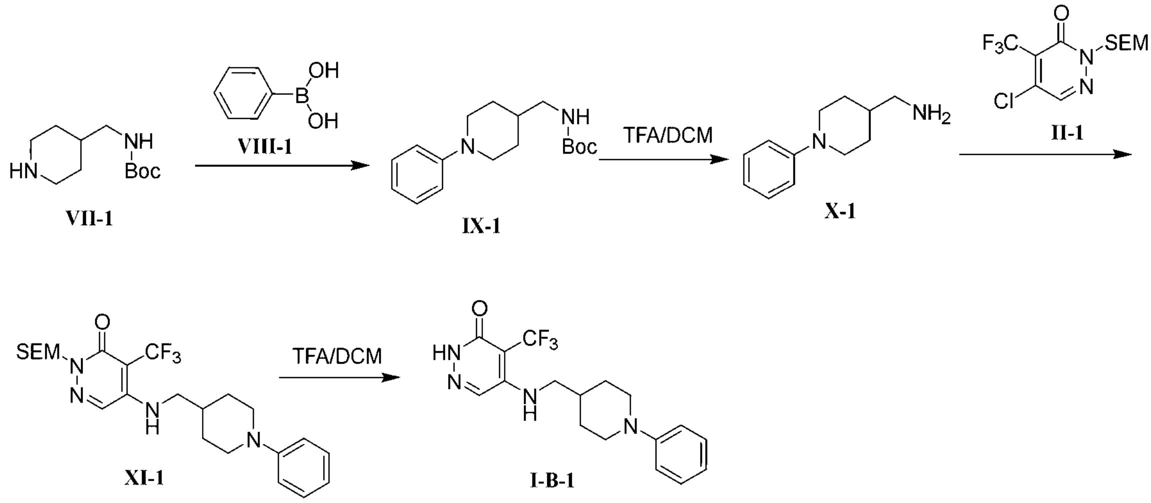


Synthesis of tert-butyl ((1-phenylpiperidin-4-yl)methyl)carbamate **(IX-1)**

4-tert-Butyloxycarbonylaminomethyl piperidine (VII-1) (500.0 mg, 2.5 mmol) was added to a 25 mL eggplant-shaped flask and dissolved in 10 mL of dichloromethane. Phenylboronic acid (VIII-1) (1.5 g, 12.2 mmol), $Cu(AcO)_2$ (978.0 mg, 4.9 mmol), and $Et_3N$ (2.5 g, 24.5 mmol) were added, and the system was incubated at room temperature for 48 h. 10 mL of water was added, and the organic layer was separated. The aqueous layer was extracted with ethyl acetate (10 mL × 3). The organic phases were combined, washed three times with saturated brine (10 mL × 3), dried over anhydrous sodium sulfate, and filtered. The filtrate was concentrated at reduced pressure. The crude product was purified by silica gel column chromatography (Vpetroleum ether: Vethyl acetate = 8:1) to give a yellow oily substance **(IX-1,** 638 mg, 90% yield). ESI-MS: $[M+H]^+$291.2.

Synthesis of (1-phenylpiperidin-4-yl)methanamine **(X-1)**

Compound **IX-1** (520.0 mg, 1.8 mmol) was added to a 50 mL eggplant-shaped flask and dissolved in 5 mL of dichloromethane. 4 mL of trifluoroacetic acid was added, and the system was incubated at room temperature for 10 min. The reaction was monitored by thin-layer chromatography (Vpetroleum ether: Vethyl acetate = 4:1) until completion. The reaction mixture was concentrated by rotary evaporation at reduced pressure to remove the solvent and the remaining trifluoroacetic acid. 10 mL of water was added, and the mixture was back-extracted with DCM (5 mL × 3). The aqueous layer was adjusted to pH 9-10 with a NaOH solution, extracted with dichloromethane (10 mL × 3), dried over anhydrous sodium sulfate, and filtered. The filtrate was concentrated by rotary evaporation at reduced pressure to remove the solvent and give a yellow oily substance (**X-1,** 265 mg, 78% yield). ESI-MS: $[M+H]^+$191.2.

Synthesis of 5-(((1-phenylpiperidin-4-yl)methyl)amino)-4-trifluoromethyl-2-((2-(trimethylsilyl) ethoxy)methyl)pyridazin-3(2*H*)-one **(XI-1)**

Compound **X-1** (234.0 mg, 1.23 mmol) was added to a 25 mL eggplant-shaped flask and dissolved in 6 mL of 1,4-dioxane, and DIPEA (477.0 mg, 3.7 mmol) and compound **II-1** (470.0 mg, 1.5 mmol) were sequentially added. The system was incubated at room temperature for 40 min. The reaction was monitored by thin-layer chromatography (Vpetroleum ether: Vethyl acetate = 2:1) until completion. 20 mL of water was added, and the mixture was extracted with ethyl acetate (10 mL × 4). The organic phases were combined, dried over anhydrous sodium sulfate, and filtered, and the filtrate was concentrated by rotary evaporation at reduced pressure to remove the solvent. The crude product was purified by silica gel column chromatography (Vpetroleum ether: Vethyl acetate = 5:1) to give a yellow solid (XI-1, 230 mg, 40% yield). ESI-MS: $[M+H]^+$483.2.

Synthesis of 5-(((1-phenylpiperidin-4-yl)methyl)amino)-4-trifluoromethylpyridazin-3(2 *H*)-one **(I-B-1)**

Compound **XI-1** (110.0 mg, 0.23 mmol) was added to a 25 mL eggplant-shaped flask and dissolved in 4 mL of dichloromethane. 3 mL of trifluoroacetic acid was added. After the addition, the system was incubated at room temperature for 15 min. The reaction was monitored by thin-layer chromatography (Vpetroleum ether: Vethyl acetate = 1:1) until completion. The mixture was concentrated by rotary evaporation at reduced pressure to remove the solvent and the remaining trifluoroacetic acid, and adjusted to pH 8 with a saturated sodium bicarbonate solution. 10 mL of water was

added, and the mixture was extracted with ethyl acetate (10 mL × 3). The organic phases were combined, dried over anhydrous sodium sulfate, and filtered, and the filtrate was concentrated by rotary evaporation at reduced pressure to remove the solvent. The crude product was purified by silica gel column chromatography (Vpetroleum ether: Vethyl acetate = 1.5:1) to give a yellow solid **(I-B-1,** 40 mg, 57% yield). ESI-MS $[M+H]^+$:353.2; $^1H$ NMR (300 MHz, DMSO-$d_6$) δ 12.55 (s, 1H), 7.96 (s, 1H), 7.05 (t, *J* = 7.7 Hz, 2H), 6.56 (d, *J* = 7.9 Hz, 2H), 6.49 (t, *J* = 7.2 Hz, 1H), 5.66 (s, 1H), 3.70-3.61 (m, 2H), 3.20-3.10 (m, 2H), 2.94-2.89 (m, 2H), 2.78 (s, 1H), 1.85-1.79 (m, 2H), 1.27-1.22 (m, 2H).

The following compound was prepared with reference to the method for preparing compound **I-B-1:**

| Compound No. | $^1H$-NMR | MS |
|---|---|---|
| Example 22 (I-B-2) | $^1H$ NMR (300 MHz, DMSO-$d_6$) δ 12.48 (s, 1H), 7.96 (s, 1H), 7.30-7.21 (m, 1H), 7.15 (t, *J* = 7.9 Hz, 2H), 6.66 (t, *J* = 7.3 Hz, 1H), 6.42 (d, *J* = 7.9 Hz, 2H), 3.82 (t, *J* = 7.5 Hz, 2H), 3.70-3.63 (m, 2H), 3.58-3.53 (m, 2H), 2.99-2.86 (m, 1H). | ESI-MS: $[M+H]^+$ 325.1 |

Example 23: Synthesis of 5-(((1,1'-biphenyl-4-yl)methyl)amino)-4-trifluoromethylpyridazin-3(2 *H*)-one (**I-C-1)**

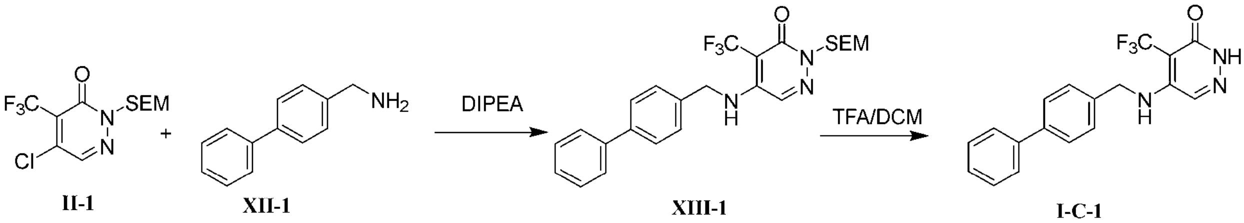


Synthesis of 5-(((1,1'-biphenyl-4-yl)methyl)amino)-4-trifluoromethyl-2-((2-(trimethylsilyl)ethoxy) methyl)pyridazin-3(2H)-one **(XIII-1)**

The compound (1,1'-biphenyl-4-yl)methylamine **(XII-1,** 155.0 mg, 0.85 mmol) was added to a 25 mL eggplant-shaped flask and dissolved in 10 mL of 1,4-dioxane. DIPEA (219.0 mg, 1.7 mmol) was added, and the system was stirred at room temperature for 15 min. Compound II-1 (404.0 mg, 1.3 mmol) was then added, and the system was incubated at room temperature for 2 h. The reaction was monitored by thin-layer chromatography (Vpetroleum ether: Vethyl acetate = 4:1) until completion. 20 mL of water was added, and the mixture was extracted with ethyl acetate (10 mL × 4). The organic phases were combined, washed three times with saturated brine (15 mL × 3), dried over anhydrous sodium sulfate, and filtered, and the filtrate was concentrated by rotary evaporation at reduced pressure to remove the solvent. The residue was purified by column chromatography (Vpetroleum ether: Vethyl acetate = 10:1) to give a pale yellow solid **(XIII-1,** 165 mg, 41% yield). ESI-MS: $[M+H]^+$184.3.

Synthesis of 5-(((1,1'-biphenyl-4-yl)methyl)amino)-4-trifluoromethylpyridazin-3(2 *H*)-one **(I-C-1)**

Compound **XIII-1** (150.0 mg, 0.3 mmol) was added to a 25 mL eggplant-shaped flask and dissolved in 4 mL of dichloromethane. 3 mL of trifluoroacetic acid was added. The system was incubated at room temperature for 15 min. The reaction was monitored by thin-layer chromatography (Vpetroleum ether: Vethyl acetate = 2:1) until completion. The mixture was concentrated by rotary evaporation at reduced pressure to remove the solvent and the remaining trifluoroacetic acid, and adjusted to pH 8 with a saturated sodium bicarbonate solution. 10 mL of water was added, and the mixture was extracted with ethyl acetate (10 mL × 3). The organic phases were combined, dried over anhydrous sodium sulfate, and filtered, and the filtrate was concentrated by rotary evaporation at reduced pressure to remove the solvent. The crude product was purified by silica gel column chromatography (*V*petroleum ether: *V*ethyl acetate = 2:1) to give a yellow solid **(I-C-1,** 110 mg, 99% yield). ESI-MS: $[M+H]^+$346.1; $^1H$ NMR (300 MHz, DMSO-$d_6$) δ 12.52 (s, 1H), 7.86-7.78 (m, 1H), 7.73 (s, 1H), 7.70-7.61 (m, 4H), 7.50-7.29 (m, 5H), 4.66 *(d, J*= 6.3 Hz, 2H).

The following compounds were prepared with reference to the method for preparing compound **I-C-1:**

| Compound No. | $^{1}$H-NMR | MS |
|---|---|---|
| Example 24 (I-C-2) | $^{1}$H NMR (300 MHz, DMSO-$d_6$) $\delta$ 12.64 (s, 1H), 7.85-7.77 (m, 1H) 7.77 (s, 1H), 7.40-7.30 (m, 4H), 7.29-7.21 (m, 3H), 7.20-7.13 (m, 1H), 4.68 (d, *J* = 5.9 Hz, 2H), 2.21 (s, 3H). | ESI-MS: $[M+H]^+$ 360.1 |
| Example 25 (I-C-3) | $^{1}$H NMR (300 MHz, DMSO-$d_6$) $\delta$ 12.45 (s, 1H), 7.83 (s, 1H), 7.31-7.24 (m, 2H), 7.23-7.17 (m, 3H), 7.15-7.09 (m, 1H), 3.42-3.33 (m, 2H), 2.61 (t, *J* = 7.7 Hz, 2H), 1.87-1.76 (m, 2H). | ESI-MS: $[M+H]^+$ 298.1 |
| Example 26 (I-C-4) | $^{1}$H NMR (300 MHz, DMSO-$d_6$) $\delta$ 12.49 (s, 1H), 7.80-7.70 (m, 1H), 7.67 (s, 1H), 7.40-7.20 (m, 5H), 4.62 (d, *J* = 6.3 Hz, 2H). | ESI-MS: $[M+H]^+$ 270.1 |
| Example 27 (I-C-5) | $^{1}$H NMR (300 MHz, DMSO-$d_6$) $\delta$ 12.50 (s, 1H), 7.81 (s, 1H), 7.46-7.41 (m, 3H), 7.35-7.21(m, 3H), 6.53 (d, *J* = 16.1 Hz, 1H), 6.37-6.28 (m, 1H), 4.18 (t, *J* = 5.7 Hz, 2H). | ESI-MS: $[M+H]^+$ 296.1 |
| Example 28 (I-C-6) | $^{1}$H NMR (300 MHz, DMSO-$d_6$) $\delta$ 12.41 (s, 1H), 7.91 (d, *J* = 7.3 Hz, 2H), 7.69 (t, *J* = 7.3 Hz, 1H), 7.57 (t, *J* = 7.5 Hz, 2H), 7.51 (s, 1H), 4.66-4.52 (m, 3H), 4.48-4.38 (m, 2H). | ESI-MS: $[M+H]^+$ 324.1 |
| Example 29 (I-C-7) | $^{1}$H NMR (300 Hz, DMSO-$d_6$) $\delta$ 12.32 (s, 1H), 7.46 (s, 1H), 7.39-7.22 (m, 5H), 5.78-5.69 (m, 1H), 4.80-4.66 (m, 1H), 4.25-4.08 (m, 4H), 3.03-2.86 (m, 1H). | ESI-MS: $[M+H]^+$ 326.1 |
| Example 30 (I-C-8) | $^{1}$H NMR (300 Hz, DMSO-$d_6$) $\delta$ 12.41 (s, 1H), 7.78-7.74 (m, 1H), 7.72-7.68 (m, 1H), 7.66-7.60 (m, 1H), 7.58-7.53 (m, 1H), 7.51 (s, 1H), 4.63-4.52 (m, 3H), 4.46-4.40 (m, 2H). | ESI-MS: $[M+H]^+$ 342.1 |
| Example 31 (I-C-9) | $^{1}$H NMR (300 Hz, DMSO-$d_6$) $\delta$ 12.32 (s, 1H), 7.46 (s, 1H), 7.42-7.34 (m, 1H), 7.22-7.16 (m, 2H), 7.11-7.03 (m, 1H), 5.88(d, *J* = 4.8 Hz, 1H), 4.81-4.75 (m, 1H), 4.22-4.08 (m, 4H), 3.03-2.92 (m, 1H). | ESI-MS: $[M+H]^+$ 344.1 |
| Example 32 (I-C-10) | $^{1}$H NMR (300 Hz, DMSO-$d_6$) $\delta$ 12.40 (s, 1H), 8.02-7.97 (m, 2H), 7.51 (s, 1H), 7.43-7.37 (m, 2H), 4.63-4.53 (m, 3H), 4.46-4.39 (m, 2H). | ESI-MS: $[M+H]^+$ 342.1 |
| Example 33 (I-C-11) | $^{1}$H NMR (400 Hz, DMSO-$d_6$) $\delta$ 12.31 (s, 1H), 7.45 (s, 1H), 7.42-7.37 (m, 2H), 7.16 (t, *J* = 8.9 Hz, 2H), 5.78 (d, *J* = 4.8 Hz, 1H), 4.78-4.74 (m, 1H), 4.23-4.13 (m, 3H), 4.10-4.03 (m, 1H), 2.99-2.89 (m, 1H). | ESI-MS: $[M+H]^+$ 344.1 |
| Example 34 (I-C-12) | $^{1}$H NMR (300 Hz, DMSO-$d_6$) $\delta$ 12.43 (s, 1H), 7.81 (d, *J* = 8.0 Hz, 2H), 7.51 (s, 1H), 7.37 (d, *J* = 7.9 Hz, 2H), 4.63-4.48 (m, 3H), 4.45-4.36 (m, 2H), 2.39 (s, 3H). | ESI-MS: $[M+H]^+$ 338.1 |
| Example 35 (I-C-13) | $^{1}$H NMR (300 Hz, DMSO-$d_6$) $\delta$ 12.41 (s, 1H), 7.89 (d, *J* = 8.9 Hz, 2H), 7.51 (s, 1H), 7.08 (d, *J* = 8.9 Hz, 2H), 4.63-4.54 (m, 2H), 4.53-4.45 (m, 1H), 4.45-4.36 (m, 2H), 3.85 (s, 3H). | ESI-MS: $[M+H]^+$ 354.1 |
| Example 36 (I-C-14) | $^{1}$H NMR (300 Hz, DMSO-$d_6$) $\delta$ 12.39 (s, 1H), 7.52 (s, 1H), 7.15-7.09 (m, 2H), 6.66-6.59 (m, 1H), 6.54-6.49 (m, 2H), 6.36 (d, *J* = 6.6 Hz, 1H), 4.68-4.59 (m, 2H), 4.34-4.23 (m, 1H), 4.09-4.01 (m, 2H). | ESI-MS: $[M+H]^+$ 311.1 |
| Example 37 (I-C-15) | $^{1}$H NMR (300 Hz, DMSO-$d_6$) $\delta$ 12.32 (s, 1H), 7.44 (s, 1H), 4.40 (t, *J* = 8.2 Hz, 2H), 3.95-3.86 (m, 2H), 3.75-3.66 (m, 1H), 2.40-2.29 (m, 1H), 1.75-1.61 (m, 4H), 1.57-1.47 (m, 1H), 1.30-0.88 (m, 6H). | ESI-MS: $[M+H]^+$ 317.2 |
| Example 38 (I-C-16) | $^{1}$H NMR (300 Hz, DMSO-$d_6$) $\delta$ 12.41 (s, 1H), 10.15 (s, 1H), 7.61 (*d, J* = 8.0 Hz, 2H), 7.51 (s, 1H), 7.32 (t, *J* = 7.8 Hz, 2H), 7.06 (t, *J* = 7.4 Hz, 1H), 4.50-4.34 (m, 4H), 3.71-3.60 (m, 1H). | ESI-MS: $[M+H]^+$ 339.1 |
| Example 39 (I-C-17) | $^{1}$H NMR (300 Hz, DMSO-$d_6$) $\delta$ 12.47 (s, 1H), 7.99-7.91 (m, 2H), 7.85 (s, 1H), 7.35 (t, *J* = 8.8 Hz, 2H), 6.92-6.80 (m, 1H), 4.31-4.17 (m, 1H), 3.60 (t, *J* = 7.7 Hz, 2H), 3.34-3.29 (m, 2H), 3.25 (t, *J* = 7.1 Hz, 2H), 2.56 (t, *J* = 5.8 Hz, 2H). | ESI-MS: $[M+H]^+$ 385.1 |

(continued)

| Compound No. | 1H-NMR | MS |
|---|---|---|
| Example 40 (I-C-18) | $^1$H NMR (400 Hz, DMSO-$d_6$) $\delta$ 12.48 (s, 1H), 8.06-8.03 (m, 2H), 7.91 (s, 1H), 7.71-7.66 (m, 1H), 7.59-7.54 (m, 2H), 4.34-4.27 (m, 1H), 3.77 (d, *J* = 6.5 Hz, 2H), 3.71-3.64 (m, 1H), 3.59-3.52 (m, 1H), 2.37-2.29 (m, 1H), 2.08-1.99 (m, 1H). | ESI-MS: $[M+H]^+$ 338.1 |
| Example 41 (I-C-19) | $^1$H NMR (300 Hz, DMSO-$d_6$) $\delta$ 12.60 (s, 1H), 8.03 (s, 1H), 8.01-7.99 (m, 2H), 7.66 (t, *J* = 7.3 Hz, 1H), 7.55 (t, *J* = 7.4 Hz, 2H), 3.82-3.64 (m, 3H), 3.45-3.35 (m, 2H), 1.91-1.82 (m, 2H), 1.73-1.58 (m, 2H). | ESI-MS: $[M+H]^+$ 352.1 |

Example 42: Synthesis of 5-((3-oxo-3-phenylpropyl)amino)-4-(trifluoromethyl)pyridazin-3(2 *H*)-one **(I-D-1)**

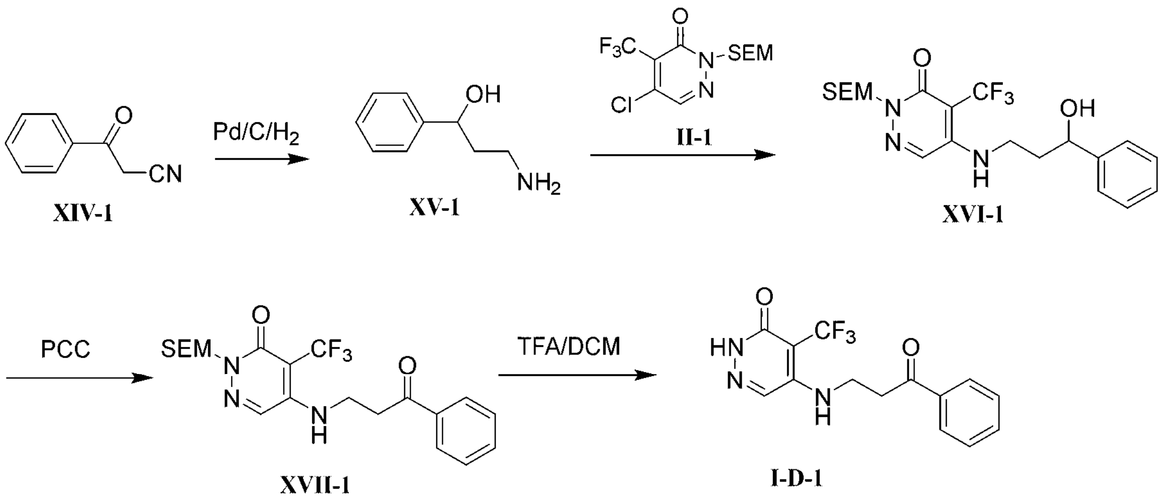


Synthesis of 3-amino-1-phenylpropan-1-ol **(XV-1)**

3-Oxo-3-phenylpropanenitrile **(XIV-1,** 1.0 g, 6.89 mmol) was added to a 100 mL three-necked flask and dissolved in 12 mL of methanol, and 10% palladium on carbon (100 mg) was added. The reaction system was purged with hydrogen 3 times and hydrogenated for 8 h. The reaction was monitored by TLC (Vdichloromethane: Vmethanol = 10:1) until completion. The reaction mixture was filtered in vacuum, and the filter cake was washed 4 times with methanol, and the filtrate and the washings were combined and concentrated by rotary evaporation at reduced pressure to remove the solvent and give a yellow oily substance (0.9 g, **XV-1),** which was directly used in the next step without purification.

Synthesis of 5-((3-hydroxy-3-phenylpropyl)amino)-4-trifluoromethyl-2-((2-(trimethylsilyl)ethoxy) methyl)pyridazin-3(2 *H*)-one **(XVI-1)**

**XV-1** (0.9 g, 5.96 mmol) was added to a 100 mL eggplant-shaped flask and dissolved in 16 mL of ethanol, and $Et_3N$ (1.8 g, 18.00 mmol) and **II-1** (2.0 g, 6.55 mmol) were added. The system was incubated at room temperature for 1 h. The reaction was monitored by TLC (*V*dichloromethane:Vmethanol = 10:1) until completion. The reaction mixture was concentrated by rotary evaporation at reduced pressure to remove the solvent, and 20 mL of water was added to the residue. The mixture was extracted with ethyl acetate (10 mL $\times$ 3). The organic phases were combined, dried over anhydrous sodium sulfate, and filtered, and the filtrate was concentrated by rotary evaporation at reduced pressure to remove the solvent. The crude product was purified by silica gel column chromatography (Vpetroleum ether: Vethyl acetate = 100:1) to give a pale yellow solid **(XVI-1,** 900 mg, 78% yield). ESI-MS: $[M+H]^+$434.2.

Synthesis of 5-((3-oxo-3-phenylpropyl)amino)-4-(trifluoromethyl)-2-((2-(trimethylsilyl)ethoxy) methyl)pyridazin-3(2 *H*)-one **(XVII-1)**

**XVI-1** (0.9 g, 2.08 mmol) was added to a 100 mL eggplant-shaped flask and dissolved in 10 mL of DCM, and PCC (1.3 g, 6.23 mmol) was added. The system was incubated at room temperature for 1 h. The reaction was monitored by thin-layer chromatography (Vdichloromethane: Vmethanol = 10:1) until completion. 20 mL of water was added. The organic phase was separated, and the aqueous layer was extracted with ethyl acetate (10 mL × 3). The organic phases were combined, dried over anhydrous sodium sulfate, and filtered, and the filtrate was concentrated by rotary evaporation at reduced pressure to remove the solvent. The crude product was purified by silica gel column chromatography (Vpetroleum ether: Vethyl acetate = 5:1) to give a yellow oily substance **(XVII-1,** 643 mg, 72% yield). ESI-MS: $[M+H]^+$432.2.

Synthesis of 5-(3-oxo-3-phenylpropylamino)-4-trifluoromethylpyridazin-3(2H)-one **(I-D-1)**

**XVII-1** (0.6 g, 1.49 mmol) was added to a 50 mL eggplant-shaped flask. Dichloromethane (5 mL) and trifluoroacetic acid (5 mL) were added. The system was incubated at room temperature for 30 min. The reaction was monitored by thin-layer chromatography (Vdichloromethane: *V*methanol = 1:1) until completion. The mixture was concentrated by rotary evaporation at reduced pressure to remove the solvent and the remaining trifluoroacetic acid, and adjusted to pH 8 with a saturated sodium bicarbonate solution. 10 mL of water was added, and the mixture was extracted with ethyl acetate (10 mL × 3). The organic phases were combined, dried over anhydrous sodium sulfate, and filtered, and the filtrate was concentrated by rotary evaporation at reduced pressure to remove the solvent. The crude product was purified by silica gel column chromatography (*V*petroleum ether: Vethyl acetate = 2:1) to give a yellow solid **(I-D-1,** 343 mg, 74% yield). ESI-MS: $[M+H]^+$312.1; $^1H$ NMR (300 MHz, DMSO-$d_6$) δ 12.48 (s, 1H), 7.99 (s, 1H), 7.98-7.96 (m, 2H), 7.67-7.62 (m, 1H), 7.55-7.49 (m, 2H), 7.04-6.97 (m, 1H), 3.74 (q, *J* = 6.6 Hz, 2H), 3.39 (t, *J* = 6.8 Hz, 2H).

The following compounds were prepared with reference to the method for preparing compound **I-D-1:**

| Compound No. | $^lH$-NMR | MS |
|---|---|---|
| Example 43 (I-D-2) | $^1H$ NMR (400 MHz, DMSO-$d_6$) δ 12.46 (s, 1H), 7.97-7.94 (m, 3H), 7.06-7.02 (m, 2H), 6.99-6.95 (m, 1H), 3.84 (s, 3H), 3.72 (q, *J* = 6.6 Hz, 2H), 3.31 (t, *J* = 6.9 Hz, 2H). | ESI-MS: $[M+H]^+$ 324.1 |
| Example 44 (I-D-3) | $^1H$ NMR (300 Hz, DMSO-$d_6$) δ 12.48 (s, 1H), 8.09-8.04 (m, 1H), 7.99-7.97 (m, 1H), 7.68-7.62 (m, 1H), 7.55-7.50 (m, 1H), 7.39-7.32 (m, 1H), 7.02-7.00 (m, 1H), 3.77-3.70 (m, 2H), 3.41-3.36 (m, 2H). | ESI-MS: $[M+H]^+$ 330.1 |
| Example 45 (I-D-4) | $^1H$ NMR (300 MHz, DMSO-$d_6$) δ 12.48 (s, 1H), 8.03-8.01 (m, 1H), 7.99-7.98 (m, 1H), 7.96 (s, 1H), 7.26-7.23 (m, 1H), 7.05-7.02 (m, 1H), 3.73 (q, *J* = 6.6 Hz, 2H), 3.31 (t, *J* = 6.8 Hz, 2H). | ESI-MS: $[M+H]^+$ 300.0 |
| Example 46 (I-D-5) | $^1H$ NMR (300 Hz, DMSO-$d_6$) δ 12.48 (s, 1H), 7.97 (s, 1H), 7.88 (d, *J*= 8.2 Hz, 2H), 7.33 (d, *J* = 8.0 Hz, 2H), 7.03-6.98 (m, 1H), 3.73 (q, *J* = 6.6 Hz, 2H), 3.35-3.32 (m, 2H), 2.37 (s, 3H). | ESI-MS: $[M+H]^+$ 326.1 |
| Example 47 (I-D-6) | $^1H$ NMR (400 Hz, DMSO-$d_6$) δ 12.47 (s, 1H), 9.00 (s, 1H),8.62 (d, *J* = 7.7 Hz, 1H), 8.09 (d, *J* = 7.8 Hz, 1H), 8.00 (s, 1H), 7.54-7.44 (m, 2H), 7.09-7.02 (m, 1H), 3.79 (q, *J* = 6.7 Hz, 2H), 3.41 (t, *J* = 6.9 Hz, 2H). | ESI-MS: $[M+H]^+$ 368.1 |
| Example 48 (I-D-7) | $^1H$ NMR (300 MHz, DMSO-$d_6$) δ 12.46 (s, 1H), 7.94 (s, 1H), 7.63-7.51 (m, 2H), 7.17-7.16 (m, 1H), 7.07-7.00 (m, 1H), 7.00-6.92 (m, 1H), 3.87 (s, 3H), 3.75-3.66 (m, 2H), 3.42-3.20 (m, 2H). | ESI-MS: $[M+H]^+$ 324.1 |
| Example 49 (I-D-8) | $^1H$ NMR (300 Hz, DMSO-$d_6$) δ 12.47 (s, 1H), 7.96 (s, 2H), 7.93 (s, 1H), 7.04-6.97 (m, 3H), 4.11 (q, *J* = 6.9 Hz, 2H), 3.72 (q, *J* = 6.4 Hz, 2H), 3.31 (t, *J* = 6.8 Hz, 2H), 1.31 (t, *J* = 7.0 Hz, 3H). | ESI-MS: $[M+H]^+$ 356.1 |
| Example 50 (I-D-9) | $^1H$ NMR (400 Hz, DMSO-$d_6$) δ 12.45 (s, 1H), 7.95 (s, 1H), 7.94 (d, *J* = 9.2 Hz, 2H), 7.03 (d, *J* = 8.8 Hz, 2H), 7.00-6.94 (m, 1H), 4.04 (d, *J* = 6.7 Hz, 2H), 3.72 (q, *J* = 6.1 Hz, 2H), 3.31 (t, *J* = 6.8 Hz, 2H), 2.78-2.66 (m, 1H), 2.11-2.03 (m, 2H), 1.95-1.77 (m, 4H). | ESI-MS: $[M+H]^+$ 396.1 |
| Example 51 (I-D-10) | $^1H$ NMR (300 Hz, DMSO-$d_6$) δ 12.47 (s, 1H), 7.95 (s, 1H), 7.92 (d, *J* = 8.8 Hz, 2H), 7.02-6.97 (m, 3H), 4.96-4.89 (m, 1H), 3.72 (q, *J* = 6.4 Hz, 2H), 3.30 (t, *J* = 6.7 Hz, 2H), 1.98-1.86 (m, 2H), 1.77-1.60 (m, 6H). | ESI-MS: $[M+H]^+$ 356.1 |
| Example 52 (I-D-11) | $^1H$ NMR (300 MHz, Chloroform-d) δ 12.47 (s, 1H), 7.95 (s, 1H), 7.93 (d, *J* = 8.0 Hz, 2H), 7.02 (d, *J* = 8.5 Hz, 2H), 7.00-6.92 (m, 1H), 3.86 (d, *J* = 5.6 Hz, 2H), 3.72 (q, *J* = 7.5 Hz, 2H), 3.30 (t, *J* = 6.4 Hz, 2H), 1.85-1.64 (m, 5H), 1.33-1.12 (m, 4H), 1.10-0.95 (m, 2H). | ESI-MS: $[M+H]^+$ 424.2 |

(continued)

| Compound No. | $^{l}$H-NMR | MS |
|---|---|---|
| Example 53 (I-D-12) | $^1$H NMR (400 Hz, DMSO-$d_6$) $\delta$ 12.45 (s, 1H), 7.95 (s, 1H), 7.93 (d, *J* = 8.7 Hz, 2H), 7.02 (d, *J* = 8.4 Hz, 2H), 6.99-6.93 (m, 1H), 3.91 (d, *J* = 7.0 Hz, 2H), 3.72 (q, *J* = 6.5 Hz, 2H), 3.30 (t, *J* = 6.6 Hz, 2H), 1.28-1.20 (m, 1H), 0.58 (q, *J* = 5.7 Hz, 2H), 0.34 (q, *J* = 5.4 Hz, 2H). | ESI-MS: $[M+H]^+$ 382.1 |
| Example 54 (I-D-13) | $^1$H NMR (300 Hz, DMSO-$d_6$) $\delta$ 12.47 (s, 1H), 7.95 (s, 1H), 7.93 (d, *J* = 8.9 Hz, 2H), 7.03 (d, *J* = 8.9 Hz, 2H), 7.00-6.94 (m, 1H), 3.93 (d, *J* = 7.0 Hz, 2H), 3.72 (q, *J* = 6.6 Hz, 2H), 3.30 (t, *J* = 6.8 Hz, 2H), 2.36-2.26 (m, 1H), 1.82-1.71 (m, 2H), 1.62-1.51 (m, 4H), 1.38-1.28 (m, 2H). | ESI-MS: $[M+H]^+$ 410.2 |
| Example 55 (I-D-14) | $^1$H NMR (300 Hz, DMSO-$d_6$) $\delta$ 12.47 (s, 1H), 8.00 (s, 1H), 7.28 (t, *J* = 7.8 Hz, 2H), 7.20-7.07 (m, 1H), 6.97-6.87 (m, 3H), 4.11 (t, *J* = 5.0 Hz, 2H), 3.79 (t, *J*= 6.1 Hz, 2H). | ESI-MS: $[M+H]^+$ 300.1 |
| Example 56 (I-D-15) | $^1$H NMR (300 Hz, DMSO-$d_6$) $\delta$ 12.57 (s, 1H), 8.05 (d, *J* = 7.0 Hz, 2H), 7.85 (s, 1H), 7.71 (t, *J* = 7.4 Hz, 1H), 7.59 (t, *J* = 7.6 Hz, 2H), 7.11-7.02 (m, 1H), 5.09 (d, *J* = 5.3 Hz, 2H). | ESI-MS: $[M+H]^+$ 298.1 |
| Example 57 (I-D-16) | $^1$H NMR (300 Hz, DMSO-$d_6$) $\delta$ 12.48 (s, 1H), 8.00 (s, 1H), 7.97 (d, *J* = 7.2 Hz, 2H), 7.64 (t, *J* = 7.3 Hz, 1H), 7.52 (t, *J* = 7.5 Hz, 2H), 3.79 (t, *J* = 6.7 Hz, 2H), 3.46 (t, *J* = 6.5 Hz, 2H), 3.02 (s, 3H). | ESI-MS: $[M+H]^+$ 326.1 |
| Example 58 (I-D-17) | $^1$H NMR (300 Hz, DMSO-$d_6$) $\delta$ 12.47 (s, 1H), 7.93 (s, 1H), 7.20-7.15 (m, 4H), 7.12-7.04 (m, 1H), 4.62 (d, *J* = 9.6 Hz, 2H), 3.69-3.59 (m, 4H), 2.85 (t, *J* = 6.0 Hz, 1H), 2.78-2.70 (m, 3H). | ESI-MS: $[M+H]^+$ 367.1 |
| Example 59 (I-D-18) | $^1$H NMR (400 Hz, DMSO-$d_6$) $\delta$ 12.48 (s, 1H), 8.07 (d, *J* = 8.7 Hz, 1H), 7.98 (s, 1H), 7.23 *(d, J* = 7.4 Hz, 1H), 7.15 (t, *J* = 7.7 Hz, 1H), 7.08-7.02 (m, 1H), 6.99 (t, *J* = 7.4 Hz, 1H), 4.07 (d, *J* = 8.5 Hz, 2H), 3.70 (q, *J* = 6.5 Hz, 2H), 3.13 (t, *J* = 8.5 Hz, 2H), 2.79 (t, *J* = 6.6 Hz, 2H). | ESI-MS: $[M+H]^+$ 353.1 |

Example 60: Inhibitory activity of compounds against PARP7 enzyme

Materials: PARP7 Chemiluminescent Assay Kit, BPS Bioscience; DMSO, Sinopharm, Nivo, PerkinElmer.

Procedures:

(1) Preparation of solutions and buffers

Preparation of 10× PBS: 720 mg of $KH_2PO_4$, 45 g of NaCl, and 5.311 g of $Na_2HPO_4 \cdot 12H_2O$ were dissolved in 500 mL of deionized water. The system was adjusted to pH 7.4, sterilized at 121 °C for 30 min, and cooled, and then preserved at 4 °C for later use.

Preparation of 1 × PBS: 10× PBS was diluted 10-fold with deionized water, i.e., 1 part of 10× PBS was diluted with 9 parts of deionized water.

Preparation of wash buffer: 1 × PBS containing 0.05% Tween-20.

Preparation of 1 × PARP buffer: (prepared right before use) 10× PARP buffer was diluted 10-fold with deionized water and placed on ice for later use.

(2) Preparation of compound working solution of different concentrations

According to the study requirements, the test compound was diluted to the desired concentration with 100% DMSO, and then diluted 10-fold with 1× PARP buffer to give a 10× compound working solution.

(3) Procedures

a. 5× histone mixture was thawed on ice the day before the study;
b. 1× histone mixture was prepared by diluting 5× histone mixture into 1× histone mixture with 1× PBS; 25 μL of 1× histone mixture was added to each well of the test plate and incubated overnight at 4 °C;
c. 100 μL of the blocking buffer was taken per well and added to the test plate, and the plate was incubated at 25 °C for 90 min;
d. After the incubation was completed, the liquid in the test plate was discarded, and the plate was washed 3 times;
e. 2.5 μL of compound working solution was taken from each well and added to the test plate according to the experimental layout; a corresponding volume of 1× PARP buffer containing 10% DMSO was added to the positive control wells (Positive control), and a corresponding volume of 1× PARP buffer was added to the blank control wells (Blank);
f. After the enzyme was completely dissolved, the enzyme stock solution was diluted to 6 ng/μL with 1× PARP buffer;
g. The enzyme solution was added to a test well plate at 10 μL/well, and a corresponding volume of 1× PARP buffer was added to the blank control wells, where the enzyme amount was 60 ng/well. Note: this procedure should be performed on ice;
h. Master mixture (12.5 μL of master mixture containing 1.25 μL of 10× PARP buffer, 1.25 μL of Opti-PARP 10× Assay mixture, and 10 μL of water) was added to the test plate at 12.5 μL/well; the test plate was sealed with a film and incubated at 25 °C for 60 min;
i. After the incubation, the liquid in the test plate was discarded, and the plate was washed 3 times;
j. Streptavidin-HRP in the kit was 50-fold diluted with the Blocking buffer, and added to the test plate at 25 μL/well, and the plate was incubated at 25 °C for 30 min;
k. After the incubation, the liquid in the test plate was discarded, and the plate was washed 3 times;
l. ELISA ECL Substrate A and ELISA ECL Substrate B in the kit were mixed in a ratio of 1:1, the mixed solution was added to the test plate at 50 μL/well, luminescence detection was immediately performed using Nivo, and the luminescence value (RLU) was read;
m. Calculation of enzyme inhibition:

$$\% \text{ Enzyme Activity} = (\text{RLU(Sample)} - \text{RLU(Blank)}) / (\text{RLU(Pos.Ctrl)} - \text{RLU(Blank)}) \times 100\%;$$

Enzyme inhibition = 1 - % Enzyme Activity; the IC 50 was calculated using GraphPad Prism 8.

The specific results are shown in Tables 1 and 2 below.

Table 1. Inhibitory activity of test compounds against PARP7 at 100 nM

| Compound No. | Inhibition at 100 nM | Compound No. | Inhibition at 100 nM |
|---|---|---|---|
| I-A-1 | +++ | I-C-14 | +++ |
| I-A-2 | +++ | I-C-17 | ++ |
| I-A-3 | ++ | I-C-18 | +++ |
| I-A-5 | +++ | I-C-19 | ++ |
| I-A-6 | ++ | I-D-1 | +++ |
| I-A-9 | ++ | I-D-2 | +++ |
| I-A-18 | ++ | I-D-3 | +++ |
| I-A-20 | +++ | I-D-5 | +++ |
| I-C-3 | ++ | I-D-6 | +++ |
| I-C-6 | +++ | I-D-7 | +++ |
| I-C-7 | +++ | I-D-8 | +++ |
| I-C-8 | +++ | I-D-9 | ++ |
| I-C-9 | +++ | I-D-10 | +++ |

(continued)

| Compound No. | Inhibition at 100 nM | Compound No. | Inhibition at 100 nM |
|---|---|---|---|
| I-C-10 | +++ | I-D-11 | +++ |
| I-C-11 | +++ | I-D-12 | ++ |
| I-C-12 | +++ | I-D-13 | +++ |
| I-C-13 | +++ | I-D-14 | +++ |
| Note: "+++" denotes that inhibition at 100 nM ≥ 50%; "++" indicates that 50% > inhibition at 100 nM ≥ 25%. | | | |

Table 2. Inhibitory activity of test compounds against PARP7 enzyme ($IC_{50}$)

| Compound No. | $IC_{50}$ | Compound No. | $IC_{50}$ |
|---|---|---|---|
| I-A-1 | +++ | I-C-14 | +++ |
| I-A-2 | +++ | I-C-18 | +++ |
| I-A-3 | ++ | I-D-1 | +++ |
| I-A-5 | ++ | I-C-12 | +++ |
| I-A-6 | ++ | I-C-13 | +++ |
| I-A-9 | ++ | I-D-2 | +++ |
| I-A-18 | ++ | I-D-3 | +++ |
| I-A-20 | +++ | I-D-5 | +++ |
| I-C-3 | ++ | I-D-6 | +++ |
| I-C-6 | +++ | I-D-7 | +++ |
| I-C-7 | +++ | I-D-8 | +++ |
| I-C-8 | +++ | I-D-10 | +++ |
| I-C-9 | +++ | I-D-11 | +++ |
| I-C-10 | +++ | I-D-13 | +++ |
| I-C-11 | +++ | I-D-14 | +++ |
| Note: "+++" denotes that $IC_{50}$ < 0.1 μM; "++" denotes that 0.1 μM ≤ $IC_{50}$ < 0.5 μM. | | | |

As shown in Tables 1 and 2, all the test compounds of the present disclosure exhibited good enzyme inhibitory activity against PARP7, and the $IC_{50}$ values of a number of compounds were less than 100 nM.

Example 61: Physicochemical properties of compounds (see Table 3; tPSA was calculated using ChemDraw Professional 17 software).

Materials: test compounds I-A-1, I-A-2, and RBN-2397.

Procedures:

(1) Caco-2 cell plating

1. 600 μL of cell culture medium and 100 μL of cell culture medium were added to the wells of a Transwell insert and a reservoir, respectively;
2. Before cell inoculation, Transwell was pre-incubated at 37 °C and 5% $CO_2$ for 1 h;
3. 100 μL of cell suspension ($4 \times 10^5$ cells/mL) was seeded into the culture medium and cultured at 37 °C, 5% $CO_2$, and 95% relative humidity for 14-21 days;
4. The cell culture medium was replaced once every other day in the first 7 days, and once every day after day 7;

5. The transepithelial electrical resistance (TEER) of the monolayer membrane was measured using EVOM3.

(2) ABBA process

1. The plate was washed twice with pre-heated HBSS (10 mM HEPES, pH 7.4) and then incubated at 37 °C for 30 min.
2. Test compounds were prepared in 1 mM DMSO and diluted 200-fold to 5 μM with HBSS (10 mM HEPES, pH 7.4) and HBSS (100 μM Lucifer Yellow, 10 mM HEPES, pH 7.4).
3. Apical to basal direction: 200 μL of 5 μM working solution (100 μM Lucifer Yellow) was added to the apical compartment, and 600 μL of HBSS (10 mM HEPES, pH 7.4) was added to the basal compartment.
4. Basal to apical direction: 600 μL of 5 μM working solution was added to the basal compartment, and 200 μL of HBSS (100 μM Lucifer Yellow, 10 mM HEPES, pH 7.4) was added to the apical compartment.
5. 100 μL of 5 μM working solution was transferred to a sample plate containing 400 μL of cold methanol, and IS* was $C_0$.
6. The cell plate was incubated at 37 °C, 5% $CO_2$, and 95% relative humidity for 2 h.
7. 5 μL of the working solution was transferred from the apical compartment and the basal compartment to the plate. After 0.5 h of incubation, the 427 nM excitation values and 536 nM emission values were read by the Lucifer Yellow leakage detector.
8. After 2 h of incubation, 100 μL of the solution was transferred from the apical and basal compartments with IS* to a sample plate containing 400 μL of cold methanol.
9. The sample plate was centrifuged at 3220 g for 40 min.
10. 100 μL of the supernatant was transferred to an assay plate containing an appropriate volume of water for LC-MS/MS analysis.

(3) Calculation

The apparent permeability coefficient ($P_{app}$, cm/s) and the efflux ratio were calculated using Microsoft Excel.

$$P_{app} = \frac{V_A \times C_{\mathbf{acceptor}} \times 100}{\text{Area} \times \text{Time} \times C_{\mathbf{initial,donor}}}$$

$$\text{Efflux Ratio} = \frac{\mathrm{P}_{\mathbf{app(B-A)}}}{\mathrm{P}_{\mathbf{app(A-B)}}}$$

Table 3. Physicochemical properties of test compounds

| Compound No. | $P_{appA\rightarrow B}$ (1E-6 cm/s) | $P_{appB\rightarrow A}$ (1E-6 cm/s) | Efflux Ratio | Number of N + O | Molecular weight | tPSA |
|---|---|---|---|---|---|---|
| RBN-2397 | 4.1 | 24 | 5.9 | 10 | 523.4 | 110.99 |
| I-A-1 | 39.86 | 20.92 | 0.52 | 4 | 361.2 | 53.49 |
| I-A-2 | 39.27 | 25.16 | 0.64 | 4 | 293.2 | 53.49 |
| Note: tPSA is a predicted value. | | | | | | |

As shown in Table 3, compounds I-A-1 and I-A-2 of the present disclosure exhibited better membrane permeability and lower efflux ratio, conforming to the Clark-Lobell rules, suggesting that the two compounds theoretically can well penetrate the blood-brain barrier and treat stroke; whereas, the existing compound RBN-2397 exhibited poor membrane permeability that failed to conform to the Clark-Lobell rules, indicating difficulties in penetrating the blood-brain barrier.

The embodiments of the technical schemes of the present disclosure have been described above by way of example. It will be appreciated that the claimed scope of the present disclosure is not limited to the embodiments described above. Any modification, equivalent replacement, improvement, and the like made by those skilled in the art without departing from the spirit and principle of the present disclosure shall fall within the claimed scope of the present application.

## Claims

**1.** A compound of formula (I), or a racemate, stereoisomer, tautomer, isotopically labeled form, solvate or pharmaceutically acceptable salt thereof:

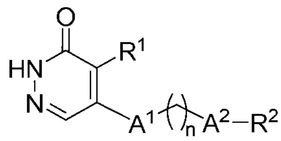

(I)

wherein:

n is selected from 0, 1, 2, 3, and 4;
$R^1$ is selected from hydrogen, halogen, cyano, nitro, $C_1$-$C_{12}$ haloalkyl, $C_1$-$C_{12}$ alkyl, $C_1$-$C_{12}$ alkyloxy, $C_1$-$C_{12}$ alkylthio, $C_1$-$C_{12}$ alkylsulfonyl, and -C(O)N($R^{11}$)($R^{12}$); $R^{11}$ and $R^{12}$ are identical or different, and are each independently selected from H and $C_1$-$C_6$ alkyl;
$A^1$ is selected from -O-, -S-,

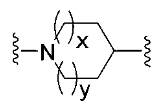

, ;

and -N($R^3$)-, wherein x and y are each independently selected from 0, 1, and 2; $R^3$ is selected from H, $C_1$-$C_{12}$ alkyl, and $C_3$-$C_{14}$ cycloalkyl;
$A^2$ is absent or selected from methylene, vinylene, ethynylene,

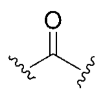
, 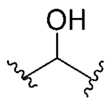
, 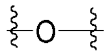
, 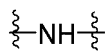
,

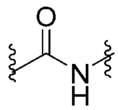
,

and the following groups unsubstituted or optionally substituted with one, two, or more $R_{a2}$:

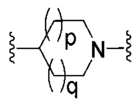
,

$C_6$-$C_{14}$ aryl, and 5- to 14-membered heteroaryl, wherein p and q are each independently selected from 0, 1, and 2; the $R_{a2}$ groups are identical or different, and are each independently selected from hydrogen, halogen, cyano, hydroxy, nitro, $C_1$-$C_{12}$ alkyl, $C_1$-$C_{12}$ alkyloxy, $C_1$-$C_{12}$ haloalkyl, $C_1$-C12 haloalkyloxy, -C(O)O$R_{a21}$, -C(O)$R_{a22}$, -N($R_{a23}$)($R_{a24}$), -S(O)$_2$$R_{a25}$, and -S(O)$R_{a26}$; the $R_{a21}$, $R_{a22}$, $R_{a23}$, $R_{a24}$, $R_{a25}$, and $R_{a26}$ groups are identical or different and are each independently selected from H, $C_1$-$C_{12}$ alkyl, and $C_1$-$C_{12}$ alkyl-C(O)-;
$R^2$ is selected from the following groups unsubstituted or optionally substituted with one, two, or more $R^{21}$: $C_6$-$C_{14}$ aryl, 5- to 14-membered heteroaryl, 3- to 8-membered heterocyclyl, $C_1$-$C_{12}$ alkyl, -S(O)$_2$-NH-, -C(O)$NH_2$, and $NH_2$-S(O)$_2$-NH-; the $R^{21}$ groups are identical or different, and are each independently selected from hydrogen, halogen, cyano, hydroxy, nitro, $C_1$-$C_{12}$ alkyl, $C_1$-$C_{12}$ haloalkyl, $C_6$-$C_{14}$ aryl, 5- to 14-membered heteroaryl, 3- to 8-membered heterocyclyl, $R_{21}$-O-, -C(O)O$R_{22}$, - C(O)$R_{23}$, -N($R_{24}$)($R_{25}$), -S(O)$_2$$R_{26}$, and -S(O)$R_{27}$; the $R_{21}$, $R_{22}$, $R_{23}$, $R_{24}$, $R_{25}$, $R_{26}$, and $R_{27}$ groups are identical or different, and are each independently selected from H, $C_1$-$C_6$ alkyl, $C_1$-$C_6$ haloalkyl, 3- to 14-membered cycloalkyl, 3- to 14-membered cycloalkyl-$C_1$-$C_6$ alkyl, $C_6$-$C_{14}$ aryl, 5- to

14-membered heteroaryl, and 3- to 8-membered heterocyclyl.

**2.** The compound, or the racemate, stereoisomer, tautomer, isotopically labeled form, solvate or pharmaceutically acceptable salt thereof according to claim 1, wherein n is selected from 0, 1, and 2; $R^1$ is selected from halogen, cyano, and trifluoromethyl.

**3.** The compound, or the racemate, stereoisomer, tautomer, isotopically labeled form, solvate or pharmaceutically acceptable salt thereof according to claim 1, wherein $A^1$ is selected from

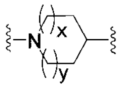

and -N($R^3$)-, wherein x and y are each independently selected from 0, 1, and 2; $R^3$ is selected from H and $C_1$-$C_6$ alkyl;

$A^1$ is selected from -NH-, -N($CH_3$)-,

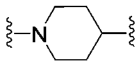, 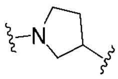 and 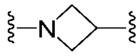;

preferably, $A^1$ is selected from -NH-;
$A^2$ is absent or selected from vinylene, ethynylene,

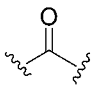, 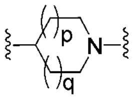, 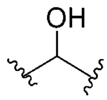, 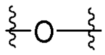, 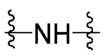,

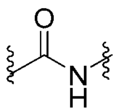,

$C_6$-$C_{10}$ arylene, and 5- to 10-membered heteroarylene, wherein p and q are each independently selected from 0, 1, and 2;
preferably, $A^2$ is absent or selected from vinylene, ethynylene, phenylene,

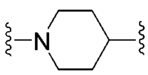, 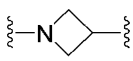,

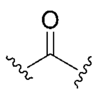, 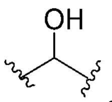, 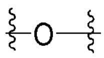, 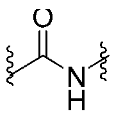, and 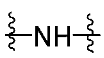;

more preferably, $A^2$ is selected from ethynylene,

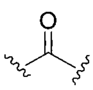, 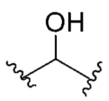, 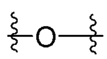, 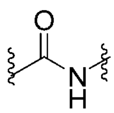,

and

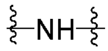;

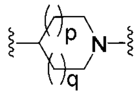

is unsubstituted or optionally substituted with one, two, or more $R_{a2}$, and the $R_{a2}$ groups are identical or different, and are each independently selected from $C_1$-$C_6$ alkyl, $C_1$-$C_6$ alkyloxy, trifluoromethyl, 2,2-difluoroethyl, methoxy, hydroxy, amino, methylamino, dimethylamino, acetylamino, carboxyl, methoxycarbonyl, methanesulfonyl, and nitro.

**4.** The compound, or the racemate, stereoisomer, tautomer, isotopically labeled form, solvate or pharmaceutically acceptable salt thereof according to claim 1, wherein $R^2$ is selected from the following groups unsubstituted or optionally substituted with one, two, or more $R^{21}$: $C_6$-$C_{10}$ aryl, 5- to 10-membered heteroaryl, and 3- to 8-membered heterocyclyl; the $R^{21}$ groups are identical or different and are each independently selected from hydrogen, halogen, $C_1$-$C_6$ alkyl, $C_1$-$C_6$ haloalkyl, $C_1$-$C_6$ alkyloxy, $C_1$-$C_6$ haloalkyloxy, 3- to 8-membered cycloalkyloxy, 3- to 8-membered cycloalkyl-$C_1$-$C_6$ alkyloxy, and $C_6$-$C_{10}$ aryl-C(O)-;

more preferably, $R^2$ is selected from substituted $C_6$-$C_{14}$ aryl and substituted 5- to 14-membered heteroaryl; the substituent is selected from hydrogen, halogen, cyano, trifluoromethyl, 2,2-difluoroethyl, $C_1$-$C_6$ alkyl, hydroxy, $C_1$-$C_6$ alkyloxy, amino, methylamino, ethylamino, dimethylamino, diethylamino, $C_3$-$C_6$ azacycloalkyl, formyl, acetyl, acetylamino, carbamoyl, and sulfamoyl; the substituent is monosubstituted or disubstituted;
even more preferably, $R^2$ is selected from substituted $C_6$-$C_{10}$ aryl and substituted 5- to 10-membered heteroaryl, wherein the $C_6$-$C_{10}$ aryl is phenyl or naphthyl, and the 5- to 10-membered heteroaryl is selected from pyridinyl, thienyl, pyrrolyl, furanyl, imidazolyl, pyrazolyl, pyrimidinyl, pyrazinyl, pyridazinyl, indolyl, quinolyl, isoquinolyl, indazolyl, benzothienyl, benzofuranyl, and benzimidazolyl; the substituent is selected from hydrogen, halogen, cyano, trifluoromethyl, 2,2-difluoroethyl, methyl, ethyl, hydroxy, methoxy, ethoxy, methylamino, ethylamino, dimethylamino, diethylamino, azetidinyl, tetrahydropyrrol-1-yl, piperidin-1-yl, morpholinyl, piperazinyl, N-methylpiperazinyl, acetyl, acetylamino, carbamoyl, and sulfamoyl; the substituent is monosubstituted or disubstituted;
still more preferably, $R^2$ is selected from

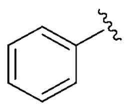 , 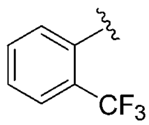 , 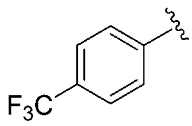 , 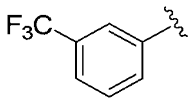 ,

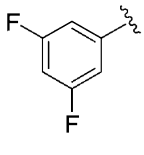 , 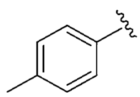 , 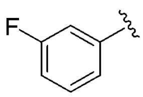 , 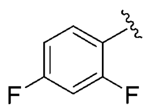 , 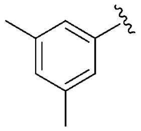 , 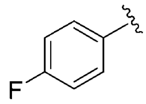 , 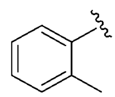 ,

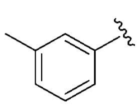 , 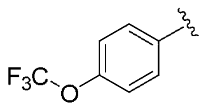 , 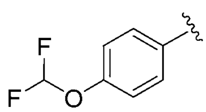 , 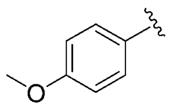 , 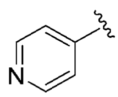 , 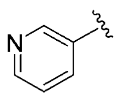 ,

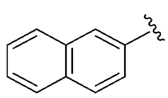 , 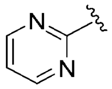 , 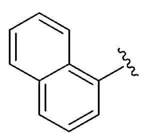 , 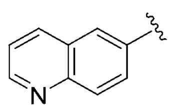 , 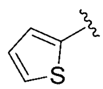 , 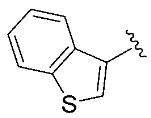 ,

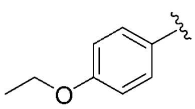 , 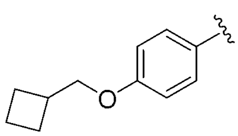 , 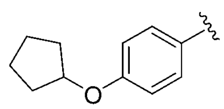 , 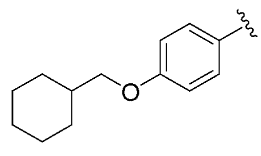 ,

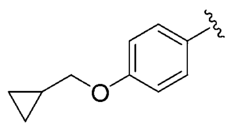, 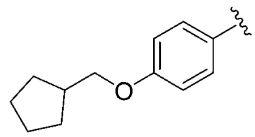, 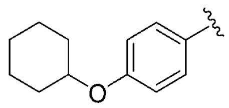, 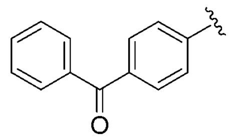,

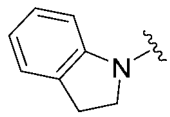, 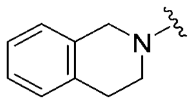, and 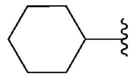.

5. The compound, or the racemate, stereoisomer, tautomer, isotopically labeled form, solvate or pharmaceutically acceptable salt thereof according to any one of claims 1-4, wherein the compound of formula (I) is selected from the following compounds:

| | | |
|---|---|---|
| 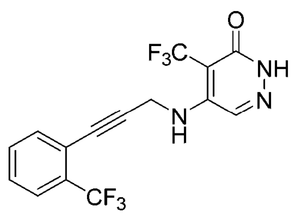 I-A-1 | 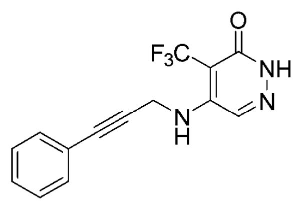 I-A-2 | 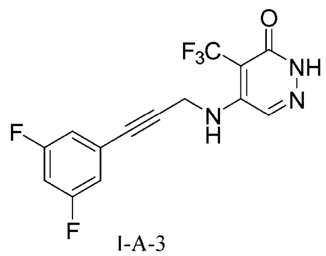 I-A-3 |
| 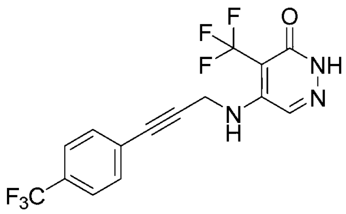 I-A-4 | 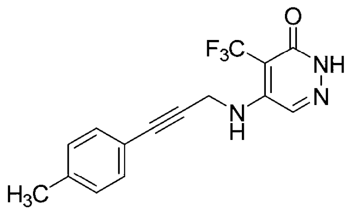 I-A-5 | 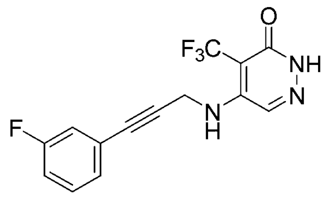 I-A-6 |
| 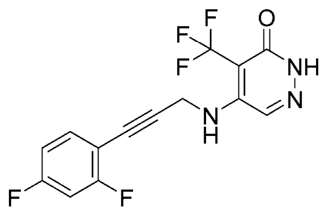 I-A-7 | 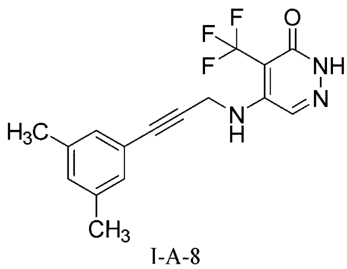 I-A-8 | 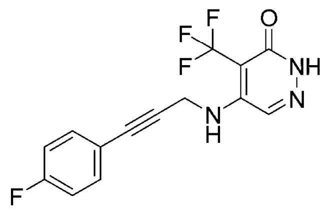 I-A-9 |
| 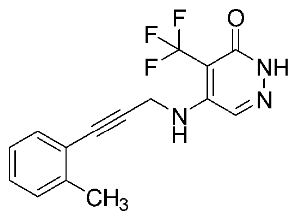 I-A-10 | 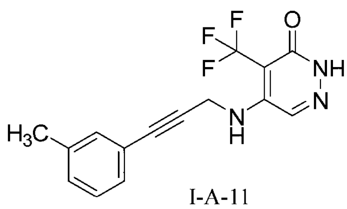 I-A-11 | 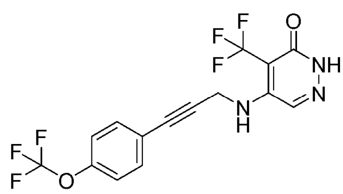 I-A-12 |

| | | |
|---|---|---|
| 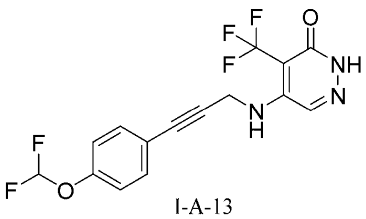 I-A-13 | 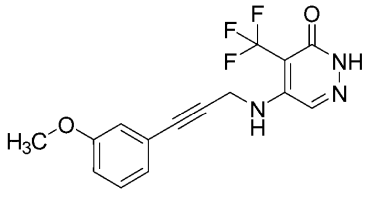 I-A-14 | 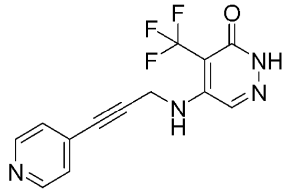 I-A-15 |
| 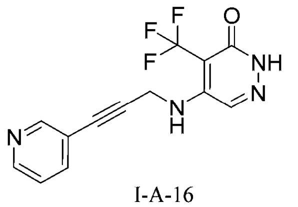 I-A-16 | 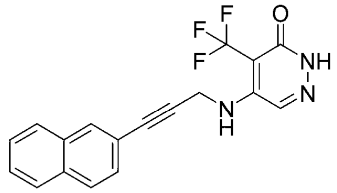 I-A-17 | 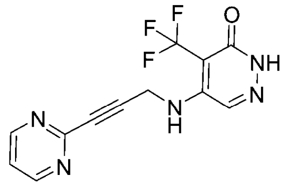 I-A-18 |
| 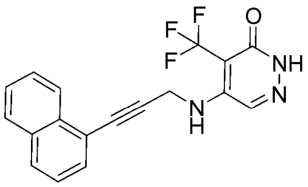 I-A-19 | 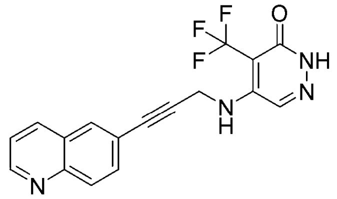 I-A-20 | 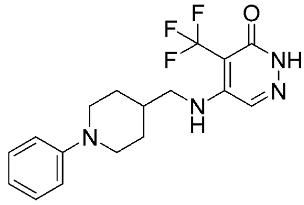 I-B-1 |
| 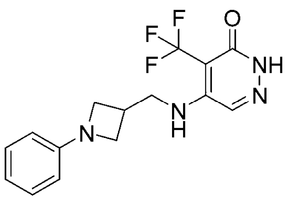 I-B-2 | 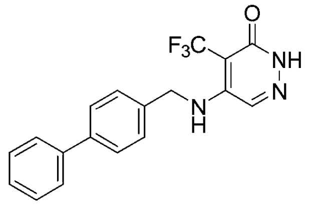 I-C-1 | 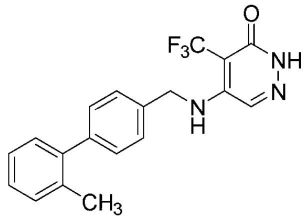 I-C-2 |
| 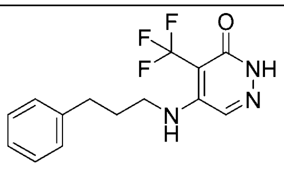 I-C-3 | 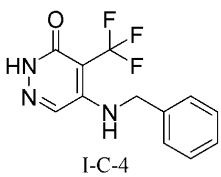 I-C-4 | 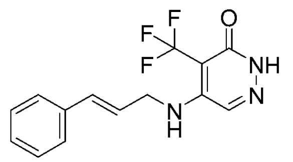 I-C-5 |
| 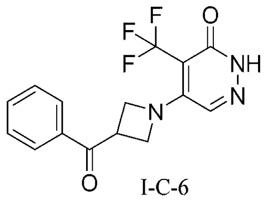 I-C-6 | 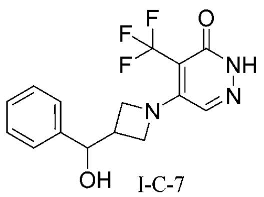 I-C-7 | 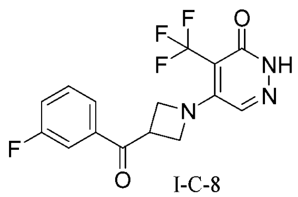 I-C-8 |

| | | |
|---|---|---|
| 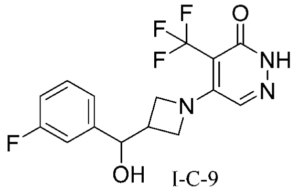 I-C-9 | 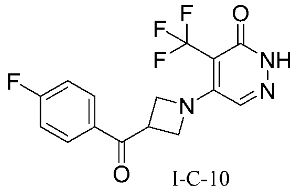 I-C-10 | 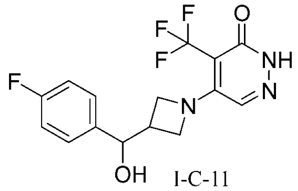 I-C-11 |
| 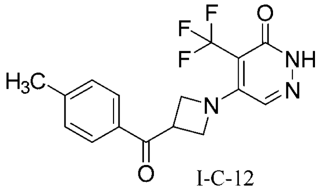 I-C-12 | 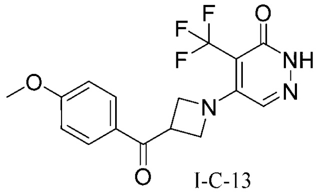 I-C-13 | 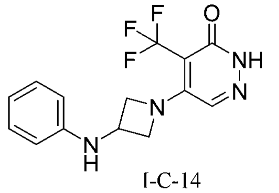 I-C-14 |
| 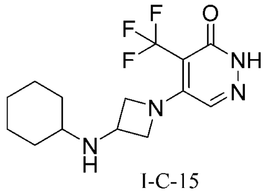 I-C-15 | 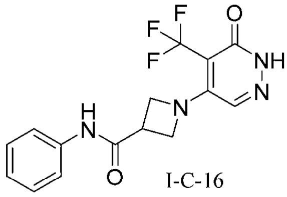 I-C-16 | 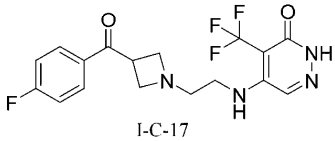 I-C-17 |
| 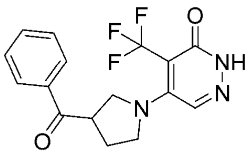 I-C-18 | 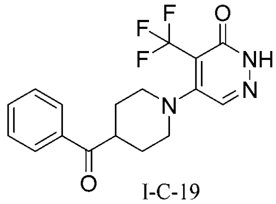 I-C-19 | 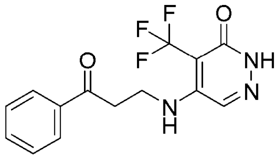 I-D-1 |
| 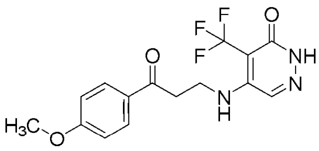 I-D-2 | 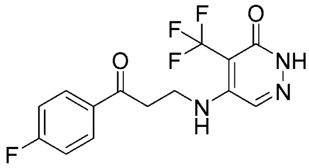 I-D-3 | 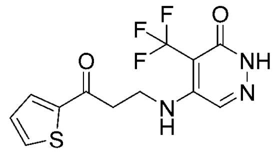 I-D-4 |
| 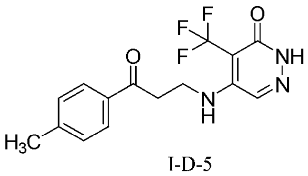 I-D-5 | 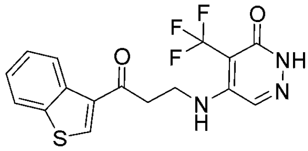 I-D-6 | 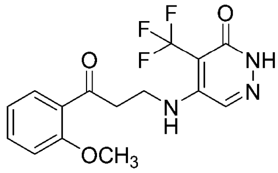 I-D-7 |

| | | |
|---|---|---|
| 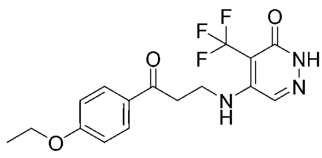 I-D-8 | 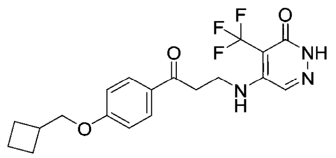 I-D-9 | 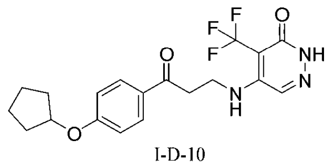 I-D-10 |
| 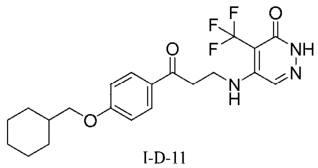 I-D-11 | 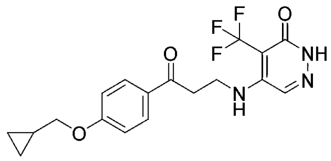 I-D-12 | 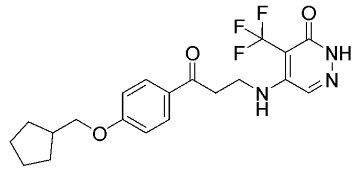 I-D-13 |
| 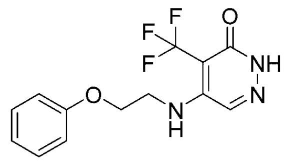 I-D-14 | 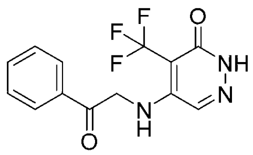 I-D-15 | 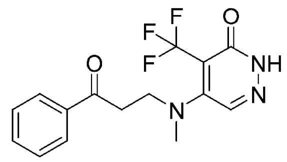 I-D-16 |
| 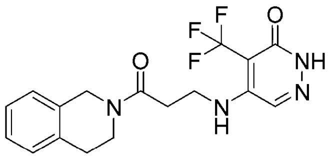 I-D-17 | 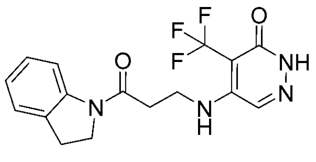 I-D-18 | . |

**6.** The compound, or the racemate, stereoisomer, tautomer, isotopically labeled form, solvate or pharmaceutically acceptable salt thereof according to claim 1, wherein the pharmaceutically acceptable salt is a salt formed by the compound and an acid, and the acid is selected from at least one of hydrochloric acid, hydrobromic acid, sulfuric acid, phosphoric acid, carbonic acid, methanesulfonic acid, benzenesulfonic acid, p-toluenesulfonic acid, naphthalenesulfonic acid, citric acid, malic acid, tartaric acid, lactic acid, pyruvic acid, acetic acid, maleic acid, succinic acid, fumaric acid, salicylic acid, phenylacetic acid, mandelic acid, and ferulic acid.

**7.** A method for preparing the compound, or the racemate, stereoisomer, tautomer, isotopically labeled form, solvate or pharmaceutically acceptable salt thereof according to any one of claims 1-6, comprising:

(1) reacting compound I-1 with compound I-2 to give compound I-3; and
(2) removing PG from compound I-3 to give the compound of formula (I);

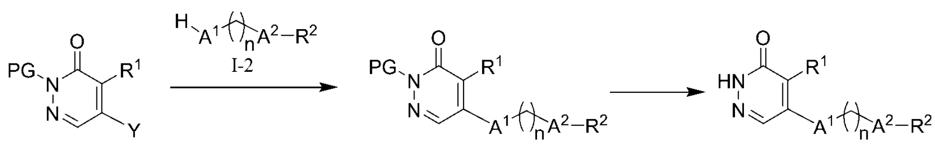


I-1 I-3 I

wherein Y is selected from a leaving group, preferably halogen, and more preferably Cl and Br; PG is selected from a protecting group, preferably (trimethylsilyl)ethoxymethyl (SEM) and p-methoxybenzyl (PMB).

**8.** A pharmaceutical composition, comprising a therapeutically effective amount of at least one of the compound, or the racemate, stereoisomer, tautomer, isotopically labeled form, solvate or pharmaceutically acceptable salt thereof according to any one of claims 1-6, and one or more pharmaceutically acceptable excipients.

**9.** Use of the compound, or the racemate, stereoisomer, tautomer, isotopically labeled form, solvate or pharmaceutically acceptable salt thereof according to any one of claims 1-6, or the pharmaceutical composition according to claim 8 in preparing a medicament for preventing and/or treating a PARP-mediated disease, preferably in preparing a PARP inhibitor medicament, wherein

more preferably, the subtype of PARP is selected from PARP2, PARP3, PARP4, PARP5a, PARP5b, PARP7, and/or PARP1.

**10.** Use of the compound, or the racemate, stereoisomer, tautomer, isotopically labeled form, solvate or pharmaceutically acceptable salt thereof according to any one of claims 1-6, or the pharmaceutical composition according to claim 8 in preparing a medicament for preventing and/or treating a central nervous system disease, a cerebral ischemia-associated disease, a cancer, an infection, an immune disease, a cardiovascular disease, or a metabolic disease, wherein the central nervous system disease is selected from stroke, preferably ischemic stroke; the cerebral ischemia-associated disease is selected from transient ischemic attack, cerebral thrombosis, and cerebral embolism; the cancer is selected from lung cancer, pancreatic cancer, colorectal cancer, leukemia, Ewing's sarcoma, breast cancer, prostate cancer, T-cell lymphoma, B-cell lymphoma, malignant rhabdomyoma, synovial sarcoma, endometrioma, gastric cancer, liver cancer, renal cancer, melanoma, ovarian cancer, cerebral glioma, cholangiocarcinoma, nasopharyngeal cancer, cervical cancer, head and neck cancer, esophageal cancer, thyroid cancer, and bladder cancer.

**INTERNATIONAL SEARCH REPORT**

International application No.
**PCT/CN2023/141928**

**A. CLASSIFICATION OF SUBJECT MATTER**

C07D 237/22(2006.01)i; C07D 403/12(2006.01)i; A61K 31/4965(2006.01)i; A61K31/497(2006.01)i; A61P 35/00(2006.01)i; A61P 25/00(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNTXT, WPABSC, ENTXTC, STN: 中国药科大学, 哒嗪酮, 哒嗪, 抑制剂, 中枢神经, 癌, 肿瘤, pyridazinone, inhibitor, cancer, tumor, PARP? , 结构检索, structure search

**C. DOCUMENTS CONSIDERED TO BE RELEVANT**

| Category* | Citation of document, with indication, where appropriate, of the relevant passages | Relevant to claim No. |
|---|---|---|
| A | CN 112424188 A (RIBON THERAPEUTICS INC.) 26 February 2021 (2021-02-26) claims 1-443, and embodiments | 1-10 |
| A | CN 114761086 A (RIBON THERAPEUTICS INC.) 15 July 2022 (2022-07-15) claims 1-80, and embodiments | 1-10 |
| A | WO 2021087018 A1 (RIBON THERAPEUTICS, INC.) 06 May 2021 (2021-05-06) claims 1-40 | 1-10 |
| A | WO 2022111700 A1 (CHENGDU BAIYU PHARMACEUTICAL CO., LTD.) 02 June 2022 (2022-06-02) claims 1-33 | 1-10 |
| A | WO 2022247839 A1 (SHANDONG XUANZHU PHARMA CO., LTD.) 01 December 2022 (2022-12-01) claims 1-13 | 1-10 |
| A | WO 2022242750 A1 (CHENGDU BAIYU PHARMACEUTICAL CO., LTD.) 24 November 2022 (2022-11-24) claims 1-26 | 1-10 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **11 March 2024** | **29 March 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/141928**

**C. DOCUMENTS CONSIDERED TO BE RELEVANT**

| Category* | Citation of document, with indication, where appropriate, of the relevant passages | Relevant to claim No. |
|---|---|---|
| A | WO 2022188889 A1 (HANGZHOU INNOGATE PHARMA CO., LTD.) 15 September 2022 (2022-09-15)<br>claims 1-16 | 1-10 |
| A | WO 2022156708 A1 (JACOBIO PHARMACEUTICALS CO., LTD.) 28 July 2022 (2022-07-28)<br>claims 1-89 | 1-10 |
| PA | WO 2023116824 A1 (CHINA PHARMACEUTICAL UNIVERSITY) 29 June 2023 (2023-06-29)<br>entire document | 1-10 |

Form PCT/ISA/210 (second sheet) (July 2022)

**INTERNATIONAL SEARCH REPORT**

International application No.
**PCT/CN2023/141928**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **10**
because they relate to subject matter not required to be searched by this Authority, namely:

Claim 10 relates to a method for treatment of diseases. In the search report, a search is carried out on the basis of the corresponding pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/141928**

| Patent document cited in search report | Publication date (day/month/year) | Patent family member(s) | Publication date (day/month/year) |
|---|---|---|---|
| CN 112424188 A | 26 February 2021 | ECSP 20069404 A | 29 January 2021 |
| | | RS 64283 B1 | 31 July 2023 |
| | | PT 3788040 T | 12 July 2023 |
| | | US 11566020 B1 | 31 January 2023 |
| | | AU 2024200566 A1 | 15 February 2024 |
| | | FI 3788040 T3 | 13 June 2023 |
| | | AR 121419 A1 | 08 June 2022 |
| | | SI 3788040 T1 | 31 July 2023 |
| | | US 2019330194 A1 | 31 October 2019 |
| | | US 10550105 B2 | 04 February 2020 |
| | | HUE 062096 T2 | 28 September 2023 |
| | | IL 278116 A | 30 November 2020 |
| | | IL 278116 B1 | 01 December 2023 |
| | | MA 52486 A | 10 March 2021 |
| | | MA 52486 B1 | 28 April 2023 |
| | | ES 2949538 T3 | 29 September 2023 |
| | | TW 202344505 A | 16 November 2023 |
| | | KR 20210014108 A | 08 February 2021 |
| | | EP 3788040 A1 | 10 March 2021 |
| | | EP 3788040 B1 | 12 April 2023 |
| | | US 2023192664 A1 | 22 June 2023 |
| | | JP 2021523104 A | 02 September 2021 |
| | | JP 6942896 B2 | 29 September 2021 |
| | | IL 308983 A | 01 January 2024 |
| | | BR 112020022006 A2 | 26 January 2021 |
| | | SG 11202010183 XA | 27 November 2020 |
| | | CY 1126132 T1 | 15 November 2023 |
| | | PH 12020551760 A1 | 28 June 2021 |
| | | US 2021024502 A1 | 28 January 2021 |
| | | US 11014913 B2 | 25 May 2021 |
| | | PL 3788040 T3 | 07 August 2023 |
| | | DK 3788040 T3 | 08 May 2023 |
| | | CR 20200518 A | 22 March 2021 |
| | | HRP 20230458 T1 | 21 July 2023 |
| | | MD 3788040 T2 | 31 October 2023 |
| | | LT 3788040 T | 26 June 2023 |
| | | MX 2020011465 A | 07 December 2020 |
| | | PE 20211382 A1 | 27 July 2021 |
| | | CO 2020013599 A2 | 21 December 2020 |
| | | EA 202092590 A1 | 08 April 2021 |
| | | US 2020123134 A1 | 23 April 2020 |
| | | US 10870641 B2 | 22 December 2020 |
| | | JP 2022017221 A | 25 January 2022 |
| | | WO 2019212937 A1 | 07 November 2019 |
| | | CA 3098585 A1 | 07 November 2019 |
| | | CL 2020002821 A1 | 19 February 2021 |
| | | TW 202014416 A | 16 April 2020 |
| | | TWI 811353 B | 11 August 2023 |
| | | AU 2019262927 A1 | 12 November 2020 |
| | | AU 2019262927 B2 | 02 November 2023 |

Form PCT/ISA/210 (patent family annex) (July 2022)

**INTERNATIONAL SEARCH REPORT**
**Information on patent family members**

International application No.
**PCT/CN2023/141928**

| Patent document cited in search report | | | Publication date (day/month/year) | Patent family member(s) | | | Publication date (day/month/year) |
|---|---|---|---|---|---|---|---|
| | | | | EP | 4234551 | A2 | 30 August 2023 |
| | | | | EP | 4234551 | A3 | 11 October 2023 |
| CN | 114761086 | A | 15 July 2022 | MX | 2022005216 | A | 02 June 2022 |
| | | | | WO | 2021087025 | A1 | 06 May 2021 |
| | | | | US | 2024034728 | A1 | 01 February 2024 |
| | | | | KR | 20220109401 | A | 04 August 2022 |
| | | | | CA | 3159188 | A1 | 06 May 2021 |
| | | | | EP | 4051384 | A1 | 07 September 2022 |
| | | | | AU | 2020375821 | A1 | 02 June 2022 |
| | | | | US | 2021130342 | A1 | 06 May 2021 |
| | | | | US | 11691969 | B2 | 04 July 2023 |
| | | | | JP | 2023501253 | A | 18 January 2023 |
| | | | | AR | 120338 | A1 | 09 February 2022 |
| | | | | IL | 292432 | A | 01 June 2022 |
| | | | | TW | 202128653 | A | 01 August 2021 |
| WO | 2021087018 | A1 | 06 May 2021 | None | | | |
| WO | 2022111700 | A1 | 02 June 2022 | None | | | |
| WO | 2022247839 | A1 | 01 December 2022 | None | | | |
| WO | 2022242750 | A1 | 24 November 2022 | KR | 20240012534 | A | 29 January 2024 |
| | | | | TW | 202246277 | A | 01 December 2022 |
| | | | | CA | 3217941 | A1 | 24 November 2022 |
| | | | | AU | 2022277493 | A1 | 23 November 2023 |
| WO | 2022188889 | A1 | 15 September 2022 | None | | | |
| WO | 2022156708 | A1 | 28 July 2022 | None | | | |
| WO | 2023116824 | A1 | 29 June 2023 | None | | | |

Form PCT/ISA/210 (patent family annex) (July 2022)